# EUROPEAN PATENT APPLICATION

(11) **EP 4 335 924 A1**
(43) Date of publication of application: **13.03.2024**
(21) Application number: 23195552.7
(22) Date of filing: 05.09.2023
(51) Int. Cl.: C12N 15/85, C12N 15/64, C12P 19/34

(54) **ULTRAPURE MINIVECTORS FOR GENE THERAPY**

(30) Priority: 08.09.2022 US 202217930652
(71) Applicant: Twister Biotech, Inc., Houston TX 77225 (US); Baylor College of Medicine, Houston, TX 77030 (US)
(72) Inventor: ZECHIEDRICH, E.Lynn, Houston, 77030 (US); FOGG, Jonathan, Houston, 77030 (US); ARAVELO-SOLIZ, Lirio, Houston, 77030 (US); CATANESE, Daniel, Houston, 77225 (US); COKER, Christopher, Houston, 77225 (US)
(74) Representative: Barker Brettell LLP

(57) **Abstract**

The present invention relates to a composition comprising an ultrapure MiniVector plus a pharmaceutically acceptable carrier, wherein a) said MiniVector being a double-stranded, supercoiled circular DNA lacking a bacterial origin of replication and lacking an antibiotic selection gene or any other plasmid selection marker; b) an expressable payload sequence; c) said MiniVector being 100-1000 bp in length excluding a length of said payload sequence; and cd said MiniVector having < 0.02% contamination by a parent plasmid DNA or recombination side-products.

## Description

### FEDERALLY SPONSORED RESEARCH STATEMENT

This invention was made with government support under grants R01GM115501 and R35GM141793 awarded by the National Institutes of Health. The government has certain rights in the invention.

### REFERENCE TO ELECTRONIC SEQUENCE LISTING

The application contains a Sequence Listing which has been submitted electronically in .XML format and is hereby incorporated by reference in its entirety. Said .XML copy, created on October 7, 2022, is named "TWISTER-Ultrapure-006US02.xml" and is 214,581 bytes in size. The sequence listing contained in this .XML file is part of the specification and is hereby incorporated by reference herein in its entirety.

### FIELD OF THE DISCLOSURE

The disclosure generally relates to methods and materials for gene therapy. More particularly, it relates to methods of making DNA MiniVectors and compositions comprising MiniVectors that are ultrapure with respect to parent plasmid and recombination side products, thus obviating many of the problems that arise from lack of purity.

### BACKGROUND OF THE DISCLOSURE

Gene therapy is a medical field that focuses on the genetic modification of cells to produce a therapeutic effect by repairing or replacing defective genetic material or introducing new genetic material. The first attempt at gene therapy was performed in 1980 by Martin Cline, but the first successful nuclear gene transfer in humans didn't occur until 1989. Since this early work, there have been thousands of clinical trials attempting various ways of delivering gene therapy to patients.

Although the concept of gene therapy appears straightforward, the process includes many inherent challenges and risks. Despite the long promise of gene therapy, successes have been limited and there have been many setbacks. Most notably, there have been multiple deaths in gene therapy clinical trials, including at least 4 deaths since 2020 in a recent trial using adeno-associated viral (AAV) vectors, considered safer than the first generation of gene therapy vectors, putting the field under close scrutiny. Some of the major challenges relating to the success of gene therapy include:

**Short-lived nature**-For gene therapy to yield a prolonged therapeutic effect, the genetic material that is introduced into target cells must remain functional and the cells containing the therapeutic must be stable. Patients often require multiple treatments for efficacy, but many vectors, particularly viral vectors, cause immune responses. After the initial dosing the patient will likely develop immunity to the virus, severely reducing the efficacy of any subsequent doses. Thus, viruses are typically not suitable to be successfully used more than once. Integrating the therapeutic DNA into the genome may result in a long-term effect, but is very difficult to control. Erroneous integration may lead to unforeseen and potentially deleterious alterations to the genome, and in some cases has resulted in cancer and even death. Therefore, integration of the therapeutic DNA into the genome should be avoided. Plasmid vectors are also prone to transgene silencing. Expression of a gene encoded on a plasmid is typically at its highest level shortly after delivery to eukaryotic cells, but this expression subsequently drops to low or even undetectable levels shortly thereafter even though the plasmid remains in the cell.

**Immune responses**-Introduction of foreign genetic material may stimulate the immune system to attack the invader. This stimulation of the immune system not only reduces gene therapy effectiveness but is also a serious safety concern. The immune system's enhanced response to viruses that it has seen before reduces the effectiveness to repeated treatments. Immune responses to the viral vector also limit the dose that can be safely delivered to the patient. The immune system is also reactive with plasmid DNA, which are rich in unmethylated dinucleotide (CpG) motifs. CpG motifs are underrepresented in eukaryotic genomes and are usually methylated. Thus, plasmids containing unmethylated CpG motifs may be recognized as foreign DNA when delivered to eukaryotic cells and trigger an immune response. DNA that is bacterial in origin also triggers immune responses through other pathways, some of which are described below, in addition to the response resulting from CpG motifs.

**Problems with viral vectors**-Viral vectors are the most commonly used type of vector in gene therapy research mainly because viruses are very efficient at delivering their genetic cargo into cells. Viruses, however, carry the risks of insertional mutagenesis, random integration into the host genome, toxicity, inflammatory responses, and lack of regulation and target specificity.

**Problems with plasmid vectors**-Plasmids do not have the same risks associated with the use of viral vectors. However, their large size precludes them from effectively entering cells and delivering their cargo. Also, plasmids contain CpG motifs and bacterial sequences that can stimulate the production of cytokines, which can induce an immune reaction. Plasmids are also prone to gene silencing, which limits their long-term efficacy. Finally, most plasmids encode a replication origin for propagation. This origin could be shared (via transduction, transfection, conjugation, or CRISPR systems that are found in -40% of all tested bacteria) among bacteria in the microbiome to activate unwanted additional genes.

**Multigene disorders**-Some commonly occurring disorders, such as heart disease, high blood pressure, Alzheimer's disease, arthritis, and diabetes, among many others, are affected by mutations in or other dysfunction or combination of multiple genes, which significantly complicates gene therapy.

**Germline changes**-Some therapies may breach the Weismann barrier (between soma and germline) potentially modifying the germline, falling afoul of regulations in countries that prohibit the latter practice.

**Insertional mutagenesis**-If the genetic material is integrated in a consequential region of the genome, for example in a tumor suppressor gene, the therapy could induce cancer. This has occurred in clinical trials for X-linked severe combined immunodeficiency (X-SCID) patients, in which hematopoietic stem cells were transduced with a corrective transgene using a retrovirus, and this led to the development of T cell leukemia in 3 of 20 patients.

**Cost**-Gene therapy is still in its developmental stage and is quite costly. Viral vectors are especially expensive to generate. Alipogene tiparvovec or Glybera, for example, at a cost of $1.6 million per patient, was reported in 2013 to be the world's most expensive drug.

Viruses have evolved to physically deliver a genetic payload into cells, and this can be readily exploited to efficiently deliver a payload sequence. This efficiency of delivery is much higher than non-viral delivery methods, e.g., plasmids. Some of the most commonly used viral vectors include adenoviruses, adeno-associated viruses, retroviruses, lentiviruses, pox viruses, alphaviruses, and herpes viruses.

Viral-based vectors have disadvantages, however. Viruses can infect more than one cell type and can infect healthy cells in addition to diseased cells. Another risk is insertion of the target gene in a wrong location in the genome. In addition, there is a small chance that viral DNA could unintentionally be introduced into the patient's reproductive cells, thus producing changes that may be passed on to children. Another concern is the possibility that transferred genes could be overexpressed, producing so much of the added gene product as to be harmful. The use of viruses is also burdened with concerns of mutation of the viral nucleic acid and subsequent uncontrolled viral reactivation. Perhaps most important, viral vectors can cause immune reactions in the host and thus can often only be delivered once because of developed immunity.

Plasmids could potentially be used instead of viral-based vectors. Plasmids are far less efficient at entering cells than viruses but have utility because they are straightforward to generate and isolate. In fact, clinical trials using intramuscular injection of "naked" DNA plasmid have occurred with some success. Unfortunately, transfection of plasmids as well as expression from plasmids has been low in comparison to viral vectors-too low to affect disease in many cases. When plasmids have been effective, the effect is short-lived (for example, two weeks was reported for a clinical trial with plasmids for cystic fibrosis treatment). Toxicity also arises because of the requirement for vehicle for effective transfection. The low transfection efficiency of plasmids necessitates higher doses with a corresponding requirement for high dose of the toxic delivery vehicle.

Numerous studies have also shown that the bacterial backbone in plasmids may elicit immune responses as well as cause reduction of transgene expression. Furthermore, the introduction of antibiotic resistance genes and the origin of replication, typically encoded on plasmids for propagation, is not allowed by some government regulatory agencies.

Thus, one of the most important objectives in gene therapy is the development of safe and effective vector systems for gene transfer to eukaryotic cells. DNA vectors, such as minicircles and MiniVectors, have been developed as an alternative to viral and plasmid DNA vectors. Minicircles and MiniVectors are circular plasmid derivatives that are almost completely devoid of bacterial sequences including the genes for selection (usually antibiotic resistance genes) and origins of DNA replication.

Minicircles and MiniVectors have been used as transgene carriers for the genetic modification of mammalian cells, with the advantage that, since they do not contain bacterial sequences, they are less likely to suffer from the well documented silencing of plasmids. Silencing of transgene expression often occurs when the transgene is carried on a plasmid that also includes bacterial sequences. Being devoid of bacterial sequences, minicircles and MiniVectors are not prone to transgene silencing and are also less likely to elicit an immune response.

The absence of bacterial sequences allows MiniVectors to potentially be much smaller than their plasmid counterparts. Smaller vectors should be more readily taken up by cells and therefore should have improved transfection efficiency. Furthermore, transfection reagents/vehicles used for cell entry are delivered according to the mass of the vector; smaller vectors require less. The use of vectors less than 1,000 base pairs (bp) is highly promising, but vectors of this small length were difficult to produce and purify in significant quantities in the past. Site-specific recombination is inhibited when the recombination sites are closely spaced and when DNA supercoiling is at normal cellular levels, and intermolecular recombination between sites on two separate plasmids becomes more favorable than bending such a short sequence on the same plasmid, leading to multimeric products (Fogg et al. 2006). An alternative approach commonly used by experimentalists is the circularization of linear DNA molecules via ligation to form minicircles. However, yields are very low and intermolecular ligation contaminants are prevalent when the linear DNA molecules short enough to generate minicircles are used.

Although superficially similar, significant differences exist between minicircles and MiniVectors. One difference lies in the method of their purification. Minicircle purification relies upon endonuclease-mediated cleavage (never 100% efficient) of the parent vector in bacteria, leading to its degradation inside the bacterial cell, and fails to separate remaining un-cleaved parent plasmid or the other recombination products from the minicircle. Indeed, a recent review (Hardee et al. (2017) provides the following comparison:

| **Type of DNA vector** | **Advantages** | **Disadvantages** |
|---|---|---|
| Minicircle | Vectors have been designed that are appropriate for mammalian mitochondrial gene therapy | Some plasmid and other DNA contaminants can remain in the final product |
| MiniVector | Smallest circular DNA vector | Not well-known in the field |
| | Most supercoiled DNA vector | |
| | Greatest purity | |
| | Naked MiniVector <1200 bp resists nebulization shear forces | |

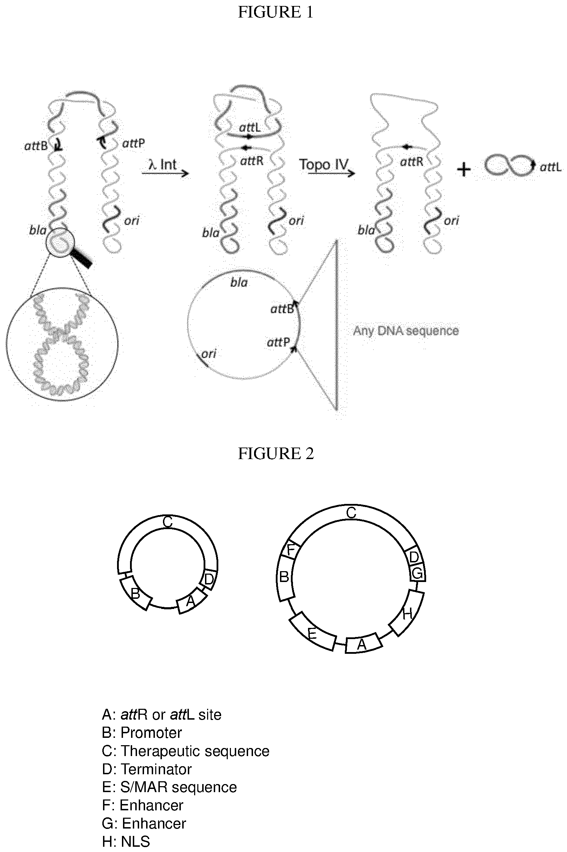

The MiniVector isolation and purification process, by contrast, solves many of the purity issues that arise with minicircles. Like minicircles, MiniVectors are synthesized from a parent plasmid via site-specific recombination, following the basic procedure shown in **FIG. 1****.** Encoding only the genetic payload and short integration sequences, MiniVectors can be engineered as small as ~250-350 bp and generated in high yields (in comparison, the smallest reported minicircle length is 650 bp, although yields of minicircles of that length are not reported). As before, unwanted bacterial sequences are on a discarded miniplasmid. However, unlike minicircles, the recombination and purification system used to make MiniVectors is highly optimized and much higher purity and yields are possible.

US7622252 overcame the problem of MiniVector yield by transforming the plasmid into a cell suitable for site-specific recombination to occur, under conditions such that topoisomerase IV decatenation activity is inhibited, thereby producing catenated DNA circles wherein at least one of the circles in each catenane is a supercoiled DNA minicircle of less than about 1 kilobase pair (kb) in size.

The different DNA species in the MiniVector purification process are typically engineered to be of sufficiently different lengths to be readily separated by size-exclusion chromatography (gel filtration). This step is a unique and major advantage of the MiniVector system and enables the recovery of a highly pure preparation of MiniVector. By contrast, a minicircle, although similarly made initially, cannot be made as small as a MiniVector and is typically less pure, carrying along up to 10% of plasmid and other DNA contaminants in the final product, which is a level of contamination well above the 1.5% allowed by some health regulatory agencies.

In addition, the MiniVector can be made with a higher degree of supercoiling by using strains having mutations to increase negative supercoiling (such as gyrase⁺ or Topoisomerase I⁻) and/or by using inhibitors of Topoisomerase I (such as seconeolitsine), and/or activators of gyrase (such as putrecine or spermadine). Supercoiling improves site specific recombination efficiency, and makes the DNA more compact, which improves transfection efficiency.

What is needed in the art are even better methods of preparing ultrapure MiniVectors, especially for use in treating patients where even minute amounts of parent plasmid may be detrimental.

### SUMMARY OF THE DISCLOSURE

This application focuses on the preparation and usage of ultrapure MiniVectors having <0.02% contamination by parent plasmid sequences, including unrecombined parent plasmid and the "miniplasmid" byproduct that contains the unwanted part of the plasmid that is not incorporated into the MiniVector. These contaminants contain bacterial sequences.

Ultrapure MiniVectors offer one or more of the following advantages over other gene delivery vehicles.
1) MiniVector transfection efficiency is equal to small interfering RNA (siRNA) and better than plasmid in several cell types tested. MiniVector DNA transfects every cell type tried, including aortic smooth muscle cells, suspension lymphoma cells, and other difficult-to-transfect cell types.
2) MiniVector knockdown efficiency lasts longer than siRNA or plasmid. Unlike therapeutically delivered RNAs, which typically degrade quickly, MiniVectors are long-lasting. Unlike plasmids, MiniVectors are not silenced.
3) Smaller therapeutic MiniVectors survive exposure to human serum for at least three times longer than a typical larger sized therapeutic plasmid (there is a strong length dependence on nuclease-mediated digestion). Finally, longer plasmids are highly susceptible to shear forces from nebulization (a possible delivery approach). Resulting degraded linear DNA can trigger DNA repair and/or activate apoptosis.
4) MiniVectors withstand shear forces associated with gene therapy delivery. For example, they survive nebulization, making them an ideal delivery vector to lungs via aerosol.
5) MiniVectors also successfully transfect T-cells, stem cells, and several types of cancer cells.

**FIG. 2** schematizes the modularity of MiniVectors. On the left is shown the simplest embodiment of a MiniVector consisting of (A) the hybrid DNA recombination sequences, *att*L or *attR,* that are products of the site-specific recombination, (B) a mammalian promoter, (C) the therapeutic DNA sequence (aka payload) to be expressed, and (D) a transcriptional terminator.

The MiniVector contains, for example, DNA encoding the therapeutic sequence expression cassette (including promoter and a sequence of interest, wherein the payload sequence may be, for example, a gene, or a segment of a gene, a sequence encoding an interfering RNA (*e*.*g*., shRNA, long hairpin RNA [lhRNA], miRNA, shRNA-embedded miRNA, long noncoding RNA [lncRNA], piwi RNA [piRNA]), or a template for *e.g.,* homology-directed repair, alteration, or replacement of the targeted DNA sequence). Importantly, the MiniVector is devoid of bacterial-originated sequences.

MiniVectors are also preferably designed to contain limited or no homology to the human genome, thus limiting the possibility of erroneous integration into the genome via homologous recombination. They are also typically much shorter in length than plasmids, further reducing the risk of integration. Therefore, the frequency of integration is at least as low as the 5 × 10⁻⁶ rate of plasmid integration and likely much lower. In contrast, many viruses are designed to integrate into the genome, and therefore, viruses present a major risk of off-target integration.

As used herein, a "MiniVector" is a double-stranded, supercoiled circular DNA typically lacking a bacterial origin of replication or an antibiotic resistance gene, or any other plasmid selection marker, much higher purity than minicircles, and have a length ~100-1000 bp, plus whatever the length of the therapeutic payload.

As used herein, "recombinant side products" include the miniplasmid that contains the unwanted bacterial sequences (origin of replication and antibiotic resistance gene or other plasmid selection marker). The miniplasmid is considered the "deletion product" following the intramolecular site-specific recombination and contains all the unwanted sequences of the original parent plasmid minus the MiniVector sequence. The miniplasmid is typically large (> 3 kb) compared to the MiniVector and is removed through the purification steps of polyethylene glycol (PEG) precipitation and gel filtration.

The recombination process that generates MiniVectors also sometimes generates double-length or even triple-length MiniVectors (or higher multimers), especially for very small MiniVectors. These multimeric forms result from intermolecular site-specific recombination between sites on two separate plasmids prior to intramolecular recombination between sites on the same plasmid. These multimers do not constitute "contaminants" because they still contain **only** the therapeutic sequence, but are merely double (or triple, etc.) the desired length. Increasing the size of the MiniVectors decreases the likelihood of multimers forming (Fogg et al. 2006). Furthermore, if a homogenous preparation of single-length MiniVector is desired, an extra gel filtration step typically separates higher multimers from single unit-sized MiniVector.

As used herein, the "payload" refers to the therapeutic sequence being delivered by the MiniVector and it can be e.g., a gene or portion thereof or an inhibitory RNA, a protein binding site, a template for DNA repair, and the like. The term "expressible payload sequence" includes the payload plus any sequences needed for mammalian expression, such as promoters, terminators, enhancers, and the like.

As used herein, "parent" sequences are the originating sequences from which the MiniVector was designed and made. A MiniVector will not have parent plasmid sequences, but just the payload itself, and its various expression components such as promoters, terminators, enhancers, and other accessory sequences such as nuclear-localization targeting sequences and the like. Each of these parent sequences can be modified to reduce CpG motifs and/or be optimized for use in humans.

As used herein, a "catenane" is a DNA architecture containing two or more circular DNA molecules linked together.

As used herein, the term "RNA interference," or "RNAi," refers to the process whereby sequence-specific, post-transcriptional gene silencing is initiated by an RNA that is homologous in sequence to the silenced gene. RNAi, which occurs in cells of a wide variety of living organisms, from plants to humans, has also been referred to as post-transcriptional gene silencing and co-suppression in different biological systems. The sequence-specific degradation of mRNA observed in RNAi is mediated by small (or short) interfering RNAs (siRNAs).

As used herein, the term "interfering RNA" means an RNA molecule capable of decreasing the expression of a gene having a nucleotide sequence at least a portion of which is substantially the same as that of the interfering RNA. As known in the art, interfering RNAs can be "small interfering RNAs," or siRNAs, composed of two complementary single-stranded RNAs that form an intermolecular duplex. Interfering RNAs can also be "short hairpin RNAs", or shRNAs, expressed as a single RNA strand that folds upon itself to form a hairpin. Interfering RNAs can also be "long hairpin RNAs," or IhRNAs, which are shRNA-like molecules with longer intramolecular duplexes and contain more than one siRNA sequence within the duplex region.

As used herein, "shRNA" is short hairpin RNA or small hairpin RNA, and "lhRNA" is long hairpin RNA, both of which can be used to silence target gene expression via RNAi.

As used herein, "miRNA" is microRNA-a small non-coding RNA molecule (containing about 22 nucleotides) found in plants, animals, and some viruses, that functions in RNA silencing and post-transcriptional regulation of gene expression. Alternative to just the duplex shRNA is an shRNA sequence embedded in a microRNA stem loop (e.g., miR-E), which may be used because it can be processed more efficiently in mammalian cells leading to more robust knockdown of the expression of the target gene. The more efficient processing of the microRNA stemloop relies on both Drosha and Dicer, whereas the contiguous duplex shRNA relies only on Dicer to cut the guide RNA that will be inserted into the RNA-induced silencing complex and RNA-induced transcriptional silencing complex.

As used herein, "lncRNA" are long non-coding RNAs. These IncRNAs are a large and diverse class of transcribed RNA molecules with a length of more than 200 nucleotides that do not encode proteins (or lack >100 amino acid open reading frame). lncRNAs are thought to encompass nearly 30,000 different transcripts in humans, hence IncRNA transcripts account for the major part of the non-coding transcriptome. lncRNA discovery is still at a preliminary stage. There are many specialized lncRNA databases, which are organized and centralized through RNAcentral (rnacentral.org). lncRNAs can be transcribed as whole or partial natural antisense transcripts to coding genes, or located between genes or within introns. Some lncRNAs originate from pseudogenes. lncRNAs may be classified into different subtypes (Antisense, Intergenic, Overlapping, Intronic, Bidirectional, and Processed) according to the position and direction of transcription in relation to other genes.

Piwi-interacting RNA or "piRNA" is the largest class of small non-coding RNA molecules expressed in animal cells. piRNAs form RNA-protein complexes through interactions with PIWI family proteins. These piRNA complexes have been linked to both epigenetic and post-transcriptional gene silencing of retrotransposons and other genetic elements in germ line cells, particularly those in spermatogenesis.

The term "treating" includes both therapeutic treatment and prophylactic treatment (reducing the likelihood of disease development). The term means decrease, suppress, attenuate, diminish, arrest, or stabilize the development or progression of a disease (*e*.*g*., a disease or disorder delineated herein), lessen the severity of the disease, or improve the symptoms associated with the disease.

MiniVectors can be delivered in a variety of "pharmaceutically acceptable" vehicles or excipients, including saline, a solvent, a branched or linear polymer (e.g., Star polymer), a liposome, a hydrogel, a lipid nanoparticle, a silicon nanoparticle, a mesoporous silica nanoparticle, a naturally occurring vesicle (e.g., an exosome), hybrids of the foregoing, or others. MiniVectors can be conjugated with a variety of ligands, agents, sugars (e.g., GalNac), nucleic acids, peptides, proteins, DNA cages, aptamers, hybrids, or other moieties to improve transfection, stability/half-life, and/or facilitate cellular or organelle localization, cell-specific, or tissue-specific uptake and/or delivery.

As described herein, the MiniVector for use in gene therapy is present in an effective amount to treat some disease. As used herein, the term "effective amount" refers to an amount which, when administered in a proper dosing regimen, is sufficient to treat (therapeutically or prophylactically) the target disorder or symptoms of the target disorder. For example, an effective amount is sufficient to reduce or ameliorate the severity, duration, or progression of the disorder being treated, prevent the advancement of the disorder being treated, cause the regression of the disorder being treated, or enhance or improve the prophylactic or therapeutic effect(s) of another therapy.

By "reducing" the expression of a target gene (and perhaps also reducing the production of a target protein), we mean a reduction of at least 10%, and preferably at least 15%, 20%, 25%, 30$%, 40% or more, but typically a complete knockout is not required, and indeed, can contribute to unwanted side effects. Ten percent might be sufficient because the person's immune response may thereby be sufficient to target and kill cancer cells, particularly in a combination therapy combined with an immune-boosting treatment, such as CpG motifs, cytokines (chemokines, interferons, interleukins, lymphokines, and tumor necrosis factors). Where a gene encodes an enzyme, we typically measure enzyme activity to determine the level of reduced expression. Otherwise, one can measure DNA or RNA or proteins levels, as appropriate for the target's function.

"Nanoparticles" are understood to comprise particles in any dimension that are less than less than 150 or more preferred less than 100 nanometers. The nanoparticle can be a viral vector, a component of a viral vector (*e*.*g*., a capsid), a non-viral vector (*e*.*g*., a plasmid or RNA or MiniVector), a cell, a fullerene and its variants, a polymer, a fat derivative (e.g., a lipid nanoparticle), an exosome, a small molecule, a peptide, metal and oxides thereof, etc.

The use of the word "a" or "an" when used in conjunction with the term "comprising" in the claims or the specification means either one or more than one, unless the context dictates otherwise. The term "about" means the stated value plus or minus the typical margin of error of measurement or plus or minus 25% if no method of measurement is indicated. The use of the term "or" in the claims is used to mean "and/or" unless explicitly indicated to refer to alternatives only or if the alternatives are mutually exclusive.

The terms "comprise," "have," "include," and "contain" (and their variants) are open-ended linking verbs and allow the addition of other elements when used in a claim. The phrase "consisting of" is closed, and excludes all additional elements. The phrase "consisting essentially of" excludes additional material elements but allows the inclusions of non-material elements that do not substantially change the nature of the invention, such as instructions for use, buffers, and the like. Any claim or claim element introduced with the open transition term "comprising," may also be narrowed to use the phrases "consisting essentially of" or "consisting of," and vice versa. However, the entirety of claim language is not repeated verbatim in the interest of brevity herein.

### BRIEF DESCRIPTION OF THE DRAWINGS

**FIG. 1****.** Generation of MiniVector DNA by λ-integrase-mediated site-specific recombination. Parent plasmid containing the sequence to be delivered flanked by *att*B and *att*P*,* the target sites for recombination. The parent plasmid is propagated in the special *E. coli* bacterial host strain, LZ54 or LZ31, harboring λ-integrase (Int) under the control of the temperature sensitive cI857 repressor. When the cells have reached a suitable density, expression of Int is switched on by a temperature shift. Recombination results in a catenated product containing the MiniVector. The products are decatenated, either by endonuclease cleavage of the large circle deletion product *ex vivo,* or by topoisomerase IV-mediated unlinking subsequent to the removal of topoisomerase inhibitor following the cell harvest. The deletion product containing the undesired bacterial sequences is removed, yielding pure, supercoiled MiniVector product. If desired, the MiniVector can encode *attR* and the deletion product can contain *att*L by switching the positions of *att*B and *att*P in the parent plasmid. *bla* = the gene encoding beta lactamase, an antibiotic resistance gene allowing for plasmid selection using ampicillin. Other plasmid selection methods may alternatively be used.
**FIG. 2****.** Modular design of MiniVectors. On the left is shown the minimal therapeutic unit, consisting only of A) *att*L or *attR* site (these sites are the products of recombination by integrase), B) a promoter, C) the therapeutic sequence (*e.g.,* **Table 1**), and D) a transcriptional terminator. The intervening regions can include any other sequence and can range in length from none to several thousand base pairs. On the right is shown a modified version containing additional modules that may be added to provide long-term persistence and expression, improve transfection, and/or facilitate nuclear localization. Any combination of these additional modules may be added to the essential modules. The exact spatial arrangement of the modules, and the distance from the therapeutic sequence, may vary from what is shown. E) S/MAR (Scaffold Matrix Attachment Region) sequences ensure that the vector is stably maintained in dividing cells. Although S/MAR sequences could potentially be placed anywhere on the MiniVector, the preferred location is upstream of the transcriptional unit to utilize the dynamic negative supercoiling generated by transcription. S/MAR function is thought to involve separation of the DNA strands, driven by the torsional strain resulting from supercoiling. F, G) Enhancer sequences may be positioned in a number of locations, depending on the identity of the enhancer. H) Nuclear localization sequences promote entry of the vector into the nucleus to facilitate expression of the therapeutic sequence. Potential sequences for these various components are listed in **Tables 2-5.**
**FIG. 3** Samples were taken during each step of PEG precipitation and anion exchange of 339 bp MiniVector. These samples were precipitated with ethanol, resuspended in TE buffer, and analyzed by agarose gel electrophoresis stained with ethidium bromide.
**FIG. 4** Gel filtration. Samples from gel filtration of a 339 bp MiniVector were analyzed by agarose gel electrophoresis stained with ethidium bromide. Larger species elute first followed by multimeric recombination products and finally the single-length MiniVector. Fractions containing single-length MiniVector were pooled and concentrated in preparation for a second pass.

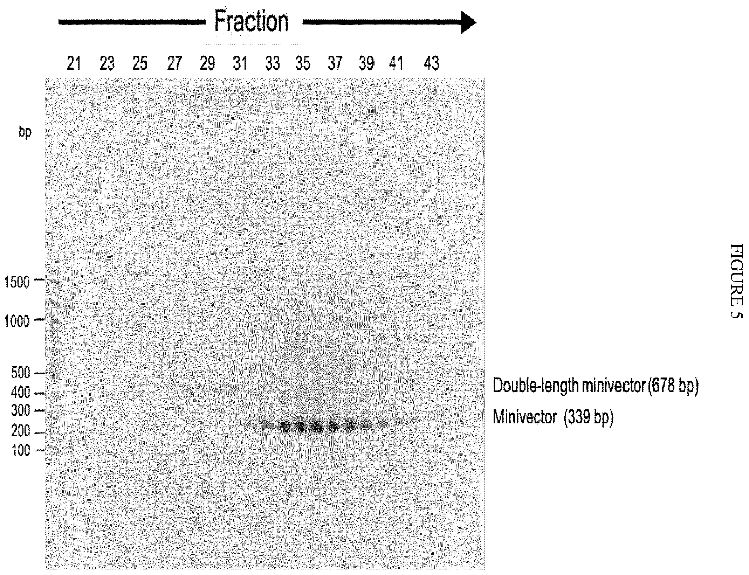

**FIG. 5****.** Gel filtration (second pass). Pooled monomeric MiniVector from the first pass were reloaded onto the gel filtration columns for a second pass. Samples from each fraction were analyzed by agarose gel electrophoresis and ethidium bromide stain.
**FIG. 6****.** Purity assessment of MiniVector using an agarose gel and SYBR Gold staining. Varying amounts of the parent plasmid were loaded to determine the lower level of sensitivity. 0.1 ng of loaded plasmid can be detected. Varying amounts of 339 bp MiniVector were loaded. Even in a 5x overloaded sample (500 ng), no parent plasmid could be detected in the lanes from the MiniVector samples, therefore plasmid contamination of this MiniVector is less than 0.1 ng in 500 ng (0.02 %).
**FIG. 7** The same gel as in FIG. 6 at 2X exposure.
**FIG. 8** The same gel as in FIG. 6 at 4X exposure.

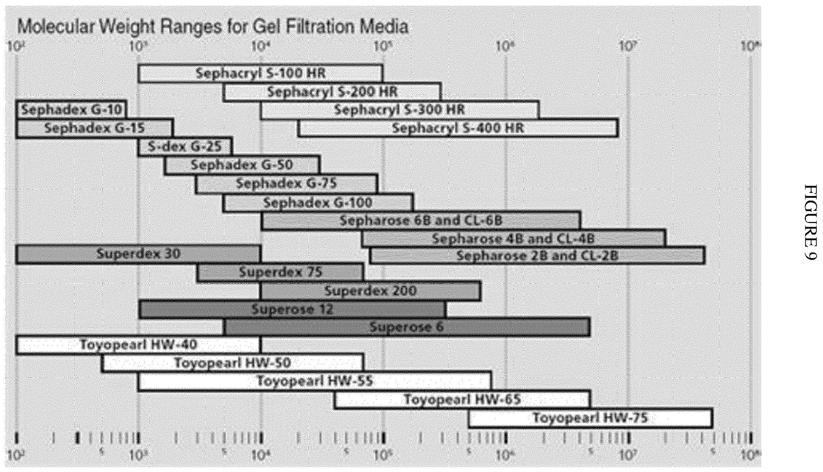

**FIG. 9** Exemplary gel filtration resins and their size exclusion separation ranges.

### DETAILED DESCRIPTION

The disclosure provides ultrapure MiniVectors that are sufficiently pure for use in gene therapy. Using the methods described herein, we demonstrate <0.02% contamination by the parent plasmid, and with more sensitive methods of analysis, we suspect that the purity is even higher (e.g., <0.01%, < 0.005% or .001%).

The invention includes any one or more of the following embodiment(s), in any combination(s) thereof:

| |
|---|
| -A composition comprising an ultrapure MiniVector plus a pharmaceutically acceptable carrier, said MiniVector being a double-stranded, supercoiled circular DNA lacking a bacterial origin of replication or an antibiotic resistance gene, or any other plasmid selection marker, having a length of about 100-1000 bp without considering the length of the therapeutic payload, and having < 0.02% contamination by a parent plasmid DNA. |
| -A MiniVector, said MiniVector being a double-stranded, supercoiled, circular DNA of at least 99.98% purity from contaminating parent plasmid DNA or recombination side products and encoding a payload that can be expressed in a mammalian cell, wherein said payload encodes an inhibitory RNA for a target gene, said target optionally selected from FOXM1, AKT, CENPA, PLK1, CDC20, BIRC5, AURKB, CCNB1, CDKN3, BCAM-AKT2, CDKN2D-WDFY2, SLC25A6, CIP2A, CD133, ALDH1A1, CD44, SALL4, MDM2, MDM4, and/or PRDM16, alone or in any combination, wherein said MiniVector lacks a bacterial origin of replication and lacks an antibiotic resistance gene, and wherein said MiniVector is made by: engineering a parent plasmid DNA molecule comprising site-specific recombination sites on either side of said expressible payload; transforming said parent plasmid into a bacterial cell suitable for site-specific recombination to occur, under conditions such that topoisomerase IV decatenation activity is inhibited, thereby producing a plurality of catenated DNA circles, wherein at least one of the circles in each catenane is a supercoiled MiniVector of less than about 2 kb in length; decatenating the catenated site-specific recombination products, thereby releasing the supercoiled MiniVector from the catenanes; and isolating the supercoiled MiniVector by PEG precipitation, anion exchange, and at least two size exclusion (gel filtration) resins each covering a different size range (e.g., progressivily reducing in size range) such that said MiniVector is at least 99.98% pure of parent plasmid or recombination side products. |
| Any MiniVector or composition comprising same, wherein contamination is assessed by gel electrophoresis and staining at a sensitivity of ≤ 0.1 ng, or preferably ≤ 0.01 ng, or wherein contamination is assessed by gel electrophoresis and staining with SYBR Gold staining at a sensitivity of ≤ 0.1 ng, or wherein contamination is assessed by gel electrophoresis, Southern blotting and probing with radiolabeled sequences that are unique to said parent DNA staining at a sensitivity of ≤ 0.01 ng. |
| Any MiniVector or composition comprising same, wherein said MiniVector is separated from said parent plasmid and recombination side-products on the basis of size, and does not use sequence-specific endonuclease cleavage *in vivo* for preparation of said MiniVector. |
| Any MiniVector or composition comprising same, wherein said MiniVector is purified by PEG precipitation of large DNA and at least two passes through multiple gel filtration columns containing different size exclusion resins, each covering a different molecular weight size range or wherein said MiniVector is purified by PEG precipitation of larger DNA species followed by anion exchange chromatography to remove RNA and non-nucleic acid components, followed by at least two passes through multiple gel filtration columns containing different size exclusion resins, each covering a different molecular weight size range, or wherein said MiniVector is purified by PEG precipitation, anion exchange chromatography, and at least two passes through multiple gel filtration columns containing different size exclusion resins, each covering a different molecular weight size range, and one or more alcohol precipitations. |
| Any MiniVector or composition comprising same, comprising a promoter operably connected to a payload operably connected to a terminator. |
| Any MiniVector or composition comprising same, wherein said payload encodes an inhibitory RNA for a target gene selected from FOXM1, AKT, CENPA, PLK1, CDC20, BIRC5, AURKB, CCNB1, CDKN3, BCAM-AKT2, CDKN2D-WDFY2, SLC25A6, CIP2A, CD133, ALDH1A1, CD44, SALL4, CHD11, MDM2, MDM4 and/or PRDM16, alone, or in combination, and |
| wherein expression of said target gene is reduced at least 10% by said inhibitory RNA when said MiniVector is introduced into mammalian cells and expressed therein; or an apoptosis gene selected from p53, p63, p73, p16, p21, p27, E2F genes, FHIT, PTEN, and/or CASPASE alone, or in combination, and said apoptosis gene is overexpressed when said MiniVector is introduced into mammalian cells. |
| Any MiniVector or composition comprising same, wherein length of the said MiniVector, outside of the payload, is <1000 bp, < 500, < 400, <300, <250, or even < 100 bp. |
| Any MiniVector or composition comprising same, wherein said payload is codon optimized for humans or human cancers, and/or wherein said MiniVector is CpG-minimized or CpG free by replacing one or more CpG dinucleotides in the MiniVector sequence, and/or wherein said MiniVector is supercoiled, and/or wherein said MiniVector has a specific DNA sequence-defined shape. |

### MINIVECTOR SYNTHESIS

MiniVector DNA was generated in bacterial cells using *in vivo* site-specific recombination as described previously in US7622252. In more detail, parent plasmids contain *att*B and *att*P (recognition sites for λ-integrase) oriented in the same direction. Site-specific recombination between the *att*B and *att*P sites exchanges the sequences between the two sites results in two product circles that are linked together (catenated) because of supercoiling in the parent plasmid. One of these product circles is the MiniVector. The other circle is the "miniplasmid", which contains the unwanted bacterial sequences. The recombination reaction also results in two new integrase sites, *att*L and attR, which are hybrid sites each containing sequences from *att*B and *att*P*.*

If attB comes first on the parent plasmid, followed by *att*P*,* with the minivector sequence in between, the larger (~180 bp) *attR* site will end up on the MiniVector and the smaller (~100 bp) *att*L site is on the miniplasmid. Conversely, if *att*P comes first, followed by *att*B*,* then the *att*L site will be on the MiniVector.

The intervening sequence between the *att*B and *att*P sites becomes incorporated into the MiniVector. Therefore, any sequence can be engineered into a MiniVector by simply cloning between the integrase sites on the parent plasmid. The system was first tested with pMC339 with *att*B preceding *att*P on the parent plasmid, which generates a 339 bp MiniVector containing an *attR* site and otherwise random sequence.

MiniVectors are generated using engineered *Escherichia coli* strains (examples include but are not limited to LZ31 and LZ54). These strains express, λ-integrase (λ-Int) under the tight control of the temperature-sensitive cI857 repressor. The *E. coli* strain is transformed with the relevant parent plasmid. When cells are grown at 30°C, no λ-Int is expressed because of the tight control afforded by the cI857 repressor. This prevents premature recombination which would result in excision of the MiniVector sequence from the parent plasmid. An aliquot of the transformed strain is grown up at 30°C in shaker flasks and used to inoculate a fermenter containing modified terrific broth medium.

Cells are grown at 30°C, maintaining the pH at 7 and the dissolved oxygen concentration above 60%, to ensure the cells remain in exponential phase. Once cells have reached mid-exponential phase, λ-Int expression is induced by shifting the culture to 43°C for ~30 minutes. The increased temperature leads to denaturation of the cI857 repressor, which prevents λ-Int expression at lower temperatures. λ-Int is not active at the higher 43°C temperature; therefore, the culture is subsequently shifted down to 30°C to allow recombination to proceed for about an hour (1-4 hrs). Prior to the temperature shift back to the lower temperature, norfloxacin is added to the fermenter prevent decatenation of the recombination products by topoisomerase IV.

### MINIVECTOR PURIFICATION

**Step 1:** The first step in purification is to harvest the cells containing MiniVector by centrifugation.

**Step 2:** Cells are first incubated with lysozyme to break down the bacterial cell walls and then lysed using a standard alkaline lysis procedure.

**Step 3:** The nucleic acid (DNA and RNA) in the lysate is precipitated with isopropanol then resuspended to reduce the volume per usual procedures. Nucleic acid solution is then incubated with RNaseA to degrade the RNA, followed by incubation with proteinase K to degrade any residual proteins.

**Step 4:** Nucleic acid solution is incubated with polyethylene glycol (PEG) and NaCl and incubated on ice for ~15 minutes. By carefully controlling the concentration of PEG, larger DNA species are selectively precipitated while the smaller minivector DNA stays in solution. For the 339 bp MiniVector exemplified herein a solution containing an equal volume of 10% PEG-8000, 1.6 M NaCl was added to the nucleic acid solution (final concentrations: 5% PEG-8000, 0.8MNaCl). For larger MiniVectors, lower concentrations of PEG are used. The precipitated larger DNA species is thus pelleted by centrifugation. The smaller nucleic acid (DNA and RNA) species in the supernatant are subsequently precipitated with ethanol to remove the PEG.

PEG precipitation is quick and has high capacity but has low resolution and can only separate DNA species significantly different in size (two-fold or more). It is used to remove the majority of the unwanted large circle (miniplasmid) recombination byproduct and any unrecombined parent plasmid. Reducing the mass of contaminating large DNA species makes subsequent downstream purification steps much more efficient.

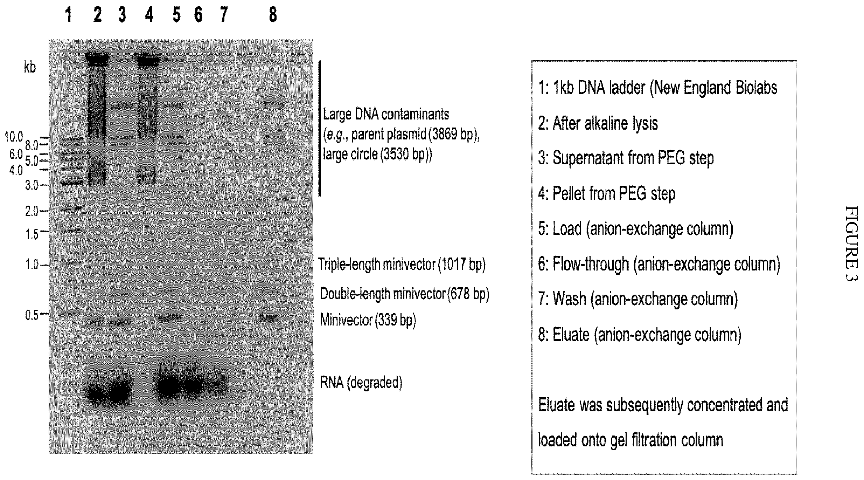

An example of the results of PEG precipitation is shown in FIG. 3 (compare the samples from the PEG pellet and the PEG supernatant). Although we have modified and optimized this step over the years, size fractionation of DNA using PEG has been extensively described previously. The innovation described here is that the MiniVectors were designed *ab initio* to be significantly smaller than the rest of the parent plasmid, allowing efficient size-dependent separation with PEG.

**Step 5:** DNA is then further purified using anion-exchange chromatography, although other methods are available. We use Qiagen plasmid purification kits for this (e.g., Maxiprep kit or Gigaprep Kit) but columns from other manufacturers may be used. The major purpose of this step is to remove the (degraded) RNA and other (non-nucleic acid) contaminants, and it does not differentiate between different sized DNA species. Following anion-exchange the DNA is again precipitated with isopropanol and resuspended in a small volume for gel filtration chromatography.

**Step 6: Gel filtration.** This step completely removes any remaining large DNA contaminants, separating DNA according to size (larger DNA species eluting first). Although described in the original US7622252 patent, we have made several modifications since that patent was filed.

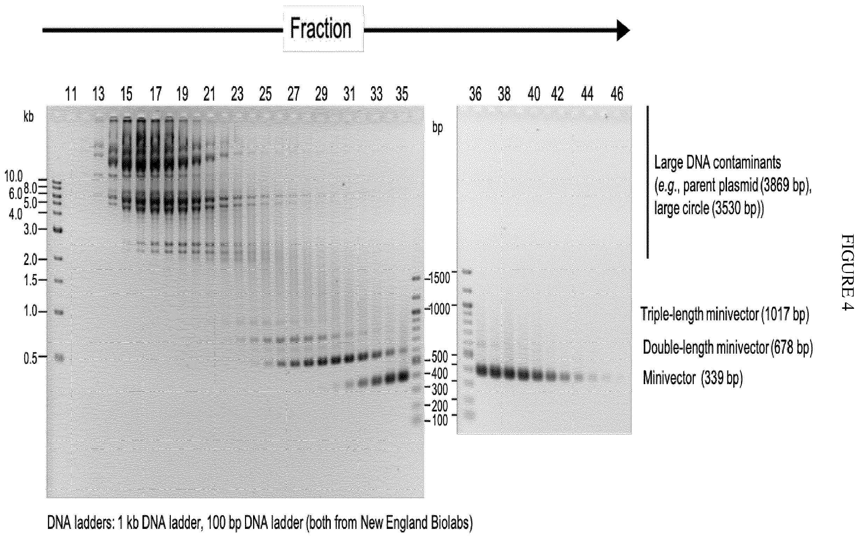

The contaminating DNA species are not able to enter the beads in the gel filtration matrix and are typically eluted in the "void volume," while the MiniVector DNA elutes later. Here, instead of using a single gel filtration column, two or three columns are connected in series such that when DNA is eluted from one column it enters the next column in the series. This significantly increases the separation of DNA species. Using multiple columns in series also allows different combinations of gel filtration resin to be used, thus optimizing size separation. For example, Sephacryl S-500 is best for separating MiniVector DNA from the parent plasmid. Sephacryl S-400 provides better separation of monomeric MiniVector from any multimeric length byproducts (see FIG. 4). Using different columns each with different size separation ranges sequentially like this allows ultrapure monomeric MiniVector to be isolated and the recovery efficiency of DNA from gel filtration is very high. Essentially all the DNA loaded onto the columns is eluted (provided that the DNA stays in solution). Therefore, there is no penalty in terms of yield for running the same DNA through the gel filtration columns multiple times. To further remove any remaining contaminants the DNA may simply be loaded again through the series of gel filtration columns.

### MINIVECTOR PURITY ASSESSMENT

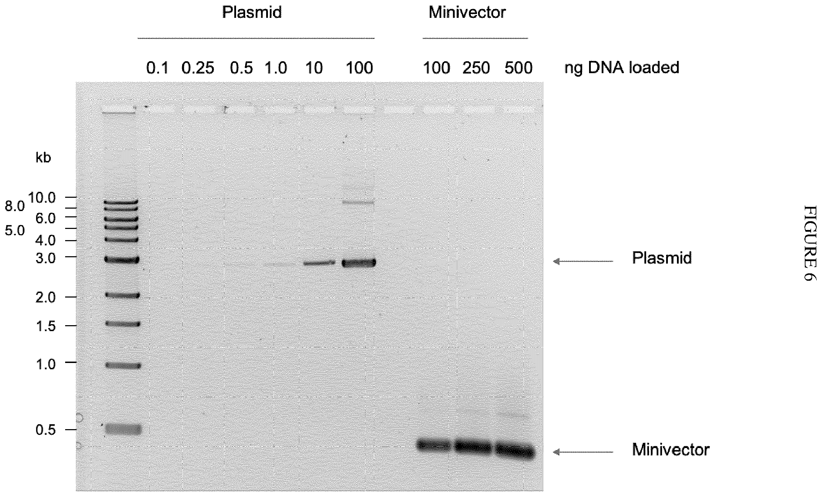

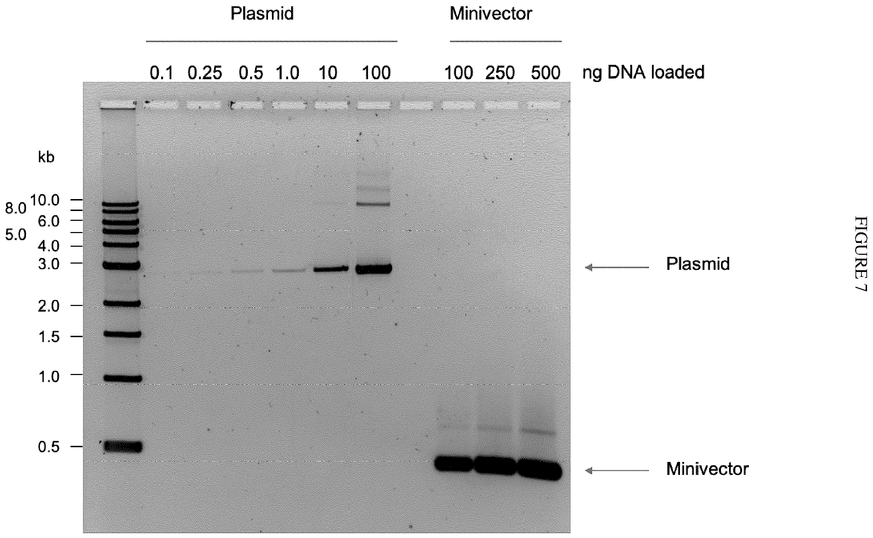

A 1% agarose gel was prepared and loaded with 1 kb DNA ladder (New England Biolabs), varying amounts of the 3.9 kb parent plasmid pMC339 and the 339 bp MiniVector (see **FIG. 6** for amounts). After running the gel at 100 V for 2 hours, it was stained with SYBR Gold and visualized using a 312 nm transilluminator on a FotoDyne FOTO/ANALYST Investigator imaging station. The gel image was captured using the imaging station CCD camera at 1X, 2X and 4X exposure levels (**FIG. 6-8**), and saved as a TIFF file. The resulting image file was analyzed and quantified using ImageQuant TL software (GE Healthcare Life Sciences).

SYBR Gold is an unsymmetrical cyanine dye that exhibits >1000-fold fluorescence enhancement upon binding to nucleic acids and has a high quantum yield (~0.6) upon binding to double- or single-stranded DNA or to RNA. Excitation maxima for dye-nucleic acid complexes are at -495 nm and ~300 nm and the emission maximum is -537 nm. This stain is ultra-sensitive-being 25-100 times more sensitive than ethidium bromide and can detect as little as 25 pg of DNA.

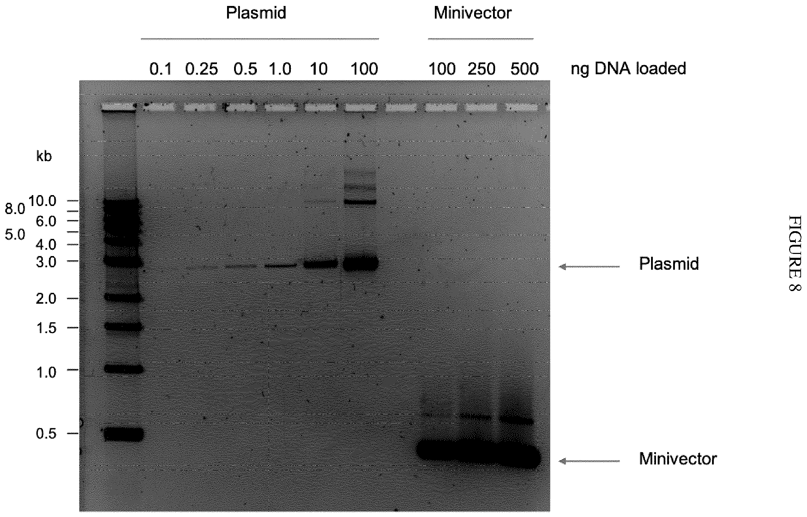

As can be seen in **FIG. 3****, zero** parent plasmid is visible in the MiniVector lanes even in a 5X overloaded gel containing 500 ng of MiniVector. Even at 4X exposure (**FIG. 8**), no band corresponding to the parent plasmid is visible. By contrast, the 0.1 ng of plasmid DNA in the plasmid sample lanes is visible, thus, parent plasmid impurity is less than 0.1 ng/500ng (< 0.02%). The minor band in the MiniVector sample lanes is double length (678 bp) MiniVector (also supercoiled). If required, this minor double-length contamination can be completely removed through an additional gel filtration step.

### MINIVECTOR BENEFITS

Numerous studies have shown that the bacterial backbone in plasmids may potentially elicit immune responses as well as cause reduction of transgene expression. CpG motifs occur approximately four times more often in bacterial and viral DNA than in the genomes of eukaryotes. These simple sequence motifs-a cytosine followed by a guanine-are recognized by Toll-like receptor 9 (TLR9), an activator of the innate immune response. Swapping the position of the bases (CpG to GpC) abolishes the immune stimulating activity. Even a single CpG motif on a vector has been reported to be enough to induce an immune response and more CpG motifs lead to a stronger immune response. Although CpG motifs are underrepresented in eukaryotic genomes they are still present but are usually methylated which mitigates their immunogenicity. Plasmids and other bacterial DNA species contain unmethylated CpG motifs and are therefore recognized as foreign DNA when delivered to eukaryotic cells, triggering an immune response. MiniVectors inherently contain fewer CpG motifs compared to the plasmids because of the removal of bacterial sequences. They can be further designed to minimize or possibly eliminate all CpG motifs altogether by modifying the therapeutic sequence encoded on the MiniVector. There are specialized software programs that can be used for this purpose. If the therapeutic sequence is a gene, software programs can also provide codon optimization to enhance the expression of the gene product in addition to minimizing CpG motifs.

Plasmids require bacterial sequences such as an origin of replication and antibiotic resistance genes in order to be propagated in bacteria during their manufacture. Another potential source of toxicity is the expression of undesired and aberrant protein products as a result of the bacterial sequences. For example, a commonly used plasmid origin of replication has been reported to contain a cryptic promoter allowing transcription to occur at sites other than the canonical promoter. This results in aberrant, undesired transcripts when the plasmid is delivered to eukaryotic cells. The expression of undesired and unexpected cryptic protein products may induce a significant immune response and has been speculated as an explanation for unforeseen adverse effects in gene therapy trials. Furthermore, the introduction of antibiotic resistance genes, often encoded on plasmids for propagation, is not allowed by some government regulatory agencies, and strongly advised against by others. Such issues are obviated by MiniVectors.

DNA transfected into eukaryotic cells is occasionally integrated into the genome of the host in approximately one cell per thousand for plasmids. Although this integration frequency is low it still poses a risk of unforeseen and potentially deleterious genetic alterations. In the worst-case scenario, integration may lead to cancer. Therefore, it is important to further mitigate any risk, particularly for a chronic disease that will require repeat dosing over an extended period of time. Integration of transfected DNA occurs through homologous recombination and illegitimate integration. The MiniVector itself is designed to contain limited or no homology to the human genome, thereby minimizing integration via homologous recombination mechanisms. It is also typically much shorter in length than plasmids, further reducing the probability of homologous recombination. Illegitimate integration requires free DNA ends, another issue that is obviated by the ultrapure supercoiled MiniVector preparations discussed herein because all the supercoiled MiniVector is comprised of circular DNA and, therefore, there are no free ends.

Shear forces associated with delivery may linearize larger vectors increasing the risk of integration. Because of their small size, MiniVectors are much more resistant to the shear forces associated with delivery including nebulization and pneumatic delivery.

The integration probability of MiniVectors is at least as low as the 5 × 10⁻⁶ rate reported for plasmid integration and likely is much lower, further adding to their benefits in terms of safety.

Since MiniVectors are small, supercoiled, lack bacterial sequences, can be CpG minimized, and are ultrapure, being free of parent plasmid and the miniplasmid deletion product from the recombination reaction, they can be successfully used in various gene therapies. These features also make these MiniVectors particularly useful for repeat gene therapy uses, e.g., long term uses (1-6 months or 1-12 months) to treat various cancers and other chronic diseases. MiniVectors preparations are also free of nicked or linear contaminants and contain a very high percentage of supercoiled DNA. In addition, the supercoiled MiniVectors more easily enter cells, and can withstand the shear forces of nebulization allowing aerosol delivery. The ability to withstand shear forces during delivery not only increases the amount of intact DNA delivered to the target cells it also has important safety benefits by limiting the generation of linear fragments. The probability of erroneous integration into the chromosome increases if the DNA is linearized. By minimizing the linearization during delivery, and without any linear DNA fragments contaminating the preparations, we avoid the problems of inducing DNA repair and recombination pathways that could lead to uncontrolled and random integration of those linear segments, as well as potential insertion or deletion of sequences of native genes because of homologous recombination or nonhomologous end joining.

### MINVECTOR PAYLOADS

Although this patent application concerns the ultrapure MiniVectors, and methods of making same, it is worth mentioning target sequences currently under development for gene therapy uses. One early payload for which we already have efficacy data is the FOXM1 shRNA (targeting 5'- ATAATTAGAGGATAATTTG-3') (SEQ ID NO: 90). Additional shRNA payloads of interest are BCAM-AKT2, CDKN2D-WDFY2, CDH11, and MDM2. Other payloads encode genes that promote apoptosis (e.g., p53, p16, p21, p27, E2F genes, FHIT, PTEN, or CASPASE).

Any such shRNAs can be designed using freely available, open access, algorithms (e.g., siRNA Wizard^{™} Software, siDESIGN Center, etc.) and then screened for off-target effects using NCBI-BLAST. Alternatively, commercially available sequences can be used for initial proof of concept work.

Note that if the therapeutic sequence is shRNA, the promoter will likely be U6 or Hl or another promoter recognized by mammalian RNA polymerase III. If said therapeutic sequence is a gene (p53, p16, p21, p27, E2F genes, PTEN, caspase, or another apoptosis inducing gene), the promoter will be CMV, EF1α, or another promoter for mammalian RNA polymerase II. **Tables 1-5** show exemplary payload and MiniVector sequences. Additional sequences and various disease targets are discussed in US Serial No. 16/180,046, incorporated by reference in its entirety for all purposes.

| **Table 1.** Payload therapeutic sequences that may be encoded on an ultrapure MiniVector | | | | | |
|---|---|---|---|---|---|
| SEQ ID NO | | Gen e | Description | Dharmacon Cat. No. | Mature Antisense |
| | 1. | AKT 2 | RAC-beta serine/threonine-protein kinase (gene AKT2) P31751 | V2LHS_237948 | AAATTCATCATCGAAGT AC |
| | 2. | | | V2LHS_132502 | TGACAAAGGTGTTGGGT CG |
| | 3. | | | V3LHS_636396 | GTGTGAGCGACTTCATC CT |
| | 4. | | | V3LHS_646518 | TGATGCTGAGGAAGAA CCT |
| | 5. | | | V3LHS_636398 | CATCATCGAAGTACCTT GT |
| | 6. | | | V3LHS_636400 | TTGATGACAGACACCTC AT |
| | 7. | | | V3LHS_325557 | TCTTTGATGACAGACAC CT |
| | 8. | ALD H1A 1 | Retinal dehydrogenase 1 P00352 | V2LHS_112035 | TTATTAAAGATGCCACG TG |
| | 9. | | | V2LHS_265598 | AAAGACAGGAAATTTCT TG |
| | 10. | | | V2LHS_112039 | ATGTCTTTGGTAAACAC TC |
| | 11. | | | V2LHS_112037 | ATCCATGTGAGAAGAAA TG |
| | 12. | | | V3LHS_398453 | ACTTTGTCTATATCCAT GT |
| | 13. | | | V3LHS_398455 | AATTCAACAGCATTGTC CA |
| | 14. | AU RK B | Aurora kinase B Q96GD4 | V2LHS_28602 | TAAGGGAACAGTTAGG GAT |
| | 15. | | | V2LHS_28606 | ATGACAGGGACCATCA GGC |
| | 16. | | | V2LHS_28601 | TTCTCCATCACCTTCTG GC |
| | 17. | | | V3LHS_341839 | TCAAGTAGATCCTCCTC CG |
| | 18. | | | V3LHS_341836 | ATGTCTCTGTGAATCAC CT |
| | 19. | | | V3LHS_341841 | TCGATCTCTCTGCGCAG CT |
| | 20. | | | V3LHS_341840 | AGAGCATCTGCCAACTC CT |
| | 21. | | | V3LHS_341837 | TTTCTGGCTTTATGTCT CT |
| | 22. | BC AM | Basal Cell Adhesion Molecule P50895 | V2LHS_62437 | ATAATGGTCGTGGGTTC CC |
| | 23. | | | V2LHS_62435 | TTGCAAACACGTTGAGC CG |
| | 24. | | | V3LHS_323253 | AATCCTCCACTCTGCAG CC |
| | 25. | | | V3LHS_323254 | TCCGCTGTCTTTAGCTC TG |
| | 26. | | | V3LHS_323256 | TGAGTGTGACTTCGTCT CC |
| | 27. | | | V3LHS_323255 | GTGACTTCGTCTCCTTC CC |
| | 28. | | | V3LHS_323251 | AGAGGTAAGGAAAGCA CCT |
| | 29. | BIR C5 | Baculoviral IAP repeat-containing protein 5 O15392 | V2LHS_94585 | ATCAAATCCATCATCTT AC |
| | 30. | | | V2LHS_94582 | TAAACAGTAGAGGAGC CAG |
| | 31. | | | V2LHS_262796 | AGCAGAAGAAACACTG GGC |
| | 32. | | | V2LHS_262484 | TTCCTAAGACATTGCTA AG |
| | 33. | | | V2LHS_230582 | TCTTGAATGTAGAGATG CG |
| | 34. | | | V3LHS_350788 | AATTCTTCAAACTGCTT CT |
| | 35. | | | V3LHS_350789 | TGTTCTTGGCTCTTTCT CT |
| | 36. | | | V3LHS_383705 | TGAAGCAGAAGAAACAC TG |
| | 37. | | | V3LHS_383704 | GAAGCAGAAGAAACACT GG |
| | 38. | CC NB1 | G2/mitotic-specific cyclin-B1 P14635 | V3LHS_369356 | TTACCATGACTACATTC TT |
| | 39. | | | V3LHS_369358 | TGCTTGCAATAAACATG GC |
| | 40. | | | V3LHS_369355 | TAATTTTCGAGTTCCTG GT |
| | 41. | | | V3LHS_369360 | AAAGCTCTTAGAATCTT CA |
| | 42. | | | V3LHS_369359 | AGAATCTTCATTTCCAT CT |
| | 43. | CD1 33 | Prominin-1 O43490 | V2LHS_71816 | ATCATTAAGGGATTGAT AG |
| | 44. | | | V2LHS_71820 | TTATACAAATCACCAAC AG |
| | 45. | | | V2LHS_71818 | TAGTAGACAATCTTTAG AC |
| | 46. | | | V2LHS_71819 | TGTTCTATAGGAAGGAC TC |
| | 47. | | | V3LHS_407402 | TTCATTTTAGAACACTT GA |
| | 48. | | | V3LHS_352745 | ATAGGAAGGACTCGTTG CT |
| | 49. | | | V3LHS_352742 | ATAGTTTCAACATCATC GT |
| | 50. | | | V3LHS_352743 | ATTATTATACAAATCACC A |
| | 51. | CD4 4 | CD44 antigen, Receptor for hyaluronic acid (HA) P16070 | V2LHS_111680 | TATATTCAAATCGATCT GC |
| | 52. | | | V2LHS_111682 | ATATGTGTCATACTGGG AG |
| | 53. | | | V2LHS_111684 | AATGGTGTAGGTGTTAC AC |
| | 54. | | | V3LHS_334831 | AGAGTTGGAATCTCCAA CA |
| | 55. | | | V3LHS_334830 | TGGGTCTCTTCTTCCAC CT |
| | 56. | | | V3LHS_334834 | TGTGCTTGTAGAATGTG GG |
| | 57. | | | V3LHS_334832 | TGTCTGAAGTAGCACTT CC |
| | 58. | CD C20 | Cell division cycle protein 20 homolog Q12834 | V2LHS_112883 | TTCCAGATGCGAATGTG TC |
| | 59. | | | V2LHS_112884 | ATAACTAGCTGGTTCTG TG |
| | 60. | | | V3LHS_640507 | AACTAGCTGGTTCTGTG CA |
| | 61. | | | V3LHS_640508 | CAGGTAATAGTCATTTC GG |
| | 62. | | | V3LHS_645717 | AAACAACTGAGGTGATG GG |
| | 63. | | | V3LHS_645716 | AATAAAAAACAACTGAG GT |
| | 64. | | | V3LHS_640514 | ACTTCCAAATAACTAGC TG |
| | 65. | | | V3LHS_363298 | TCTGCTGCTGCACATCC CA |
| | 66. | CD KN2 D | Cyclin-dependent kinase 4 inhibitor D P55273 | V2LHS_262156 | AATAAATAGAATCCATTT C |
| | 67. | | | V3LHS_401207 | ATGAATAACTCATAACT CA |
| | 68. | | | V3LHS_310385 | CCACTAGGACCTTCAG GGT |
| | 69. | | | V3LHS_310386 | CGGGATGCACCAGCTC GCG |
| | 70. | | | V3LHS_310389 | AGGACCTTCAGGGTGT CCA |
| | 71. | | | V3LHS_310387 | GAACTGCCAGATGGATT GG |
| | 72. | CD KN3 | Cyclin-dependent kinase inhibitor 3 Q16667 | V2LHS_262397 | TATAGTAGGAGACAAGC AG |
| | 73. | | | V2LHS_201585 | TGCTTGATGGTCTGTAT TG |
| | 74. | | | V3LHS_386043 | TGATTGTGAATCTCTTG AT |
| | 75. | | | V3LHS_386040 | ATCTTGATACAGATCTT GA |
| | 76. | | | V3LHS_386041 | TGATACAGATCTTGATT GT |
| | 77. | CE NP A | Histone H3-like centromeric protein A P49450 | V2LHS_150535 | ATATGATGGAAATGCCC AG |
| | 78. | | | V2LHS_150534 | TATTACCTCTGTTACAG AG |
| | 79. | | | V2LHS_150531 | TAACACATATTTCTCTTG C |
| | 80. | | | V3LHS_403419 | AAAGCAACACACACATA CT |
| | 81. | | | V3LHS_403420 | AGACTGACAGAAACACT GG |
| | 82. | | | V3LHS_403421 | TGTCTCATATATTACCT CT |
| | 83. | | | V3LHS_403422 | TATCTGAAAATTATTTTC A |
| | 84. | | | V3LHS_313522 | TTGGGAAGAGAGTAACT CG |
| | 85. | CIP 2A | CIP2A (gene KIAA1524) Q8TCG1 | V2LHS_206422 | TACTCAATGTCTTTATGT G |
| | 86. | | | V3LHS_308568 | TGAATGTGATCTATCAG GA |
| | 87. | | | V3LHS_308569 | TGTTCTCTATTATCTGA CG |
| | 88. | | | V3LHS_308565 | TTCATTTCATATACATCC A |
| | 89. | | | V3LHS_308566 | TGAACAGAAAGATTGTG CC |
| | 90. | FO XM 1 | Forkhead box protein M1 Q08050 | V2LHS_283849 | ATAATTAGAGGATAATT TG |
| | 91. | | | V3LHS_396939 | ATTGTTGATAGTGCAGC CT |
| | 92. | | | V3LHS_396937 | TGAATCACAAGCATTTC CG |
| | 93. | | | V3LHS_396941 | TGATGGTCATGTTCCGG CG |
| | 94. | | | V3LHS_396940 | AATAATCTTGATCCCAG CT |
| | 95. | PLK 1 | Serine/threonine-protein kinase PLK1 P53350 | V2LHS_19709 | ATTCTGTACAATTCATAT G |
| | 96. | | | V2LHS_19711 | ATAGCCAGAAGTAAAGA AC |
| | 97. | | | V2LHS_241437 | TGCGGAAATATTTAAGG AG |
| | 98. | | | V2LHS_19708 | GTAATTAGGAGTCCCAC AC |
| | 99. | | | V2LHS_262328 | AATTAGGAGTCCCACAC AG |
| | 100. | | | V3LHS_311459 | TTCTTGCTCAGCACCTC GG |
| | 101. | | | V3LHS_311462 | TTGACACTGTGCAGCTG CT |
| | 102. | | | V3LHS_311463 | TAGGCACAATCTTGCCC GC |
| | 103. | PR DM 16 | PR domain zinc finger protein 16 Q9HAZ2 | V2LHS_215636 | TAAAGCCTCAGAATCTA AG |
| | 104. | | | V2LHS_251390 | TAAATTACGACTCTGAC AC |
| | 105. | | | V3LHS_300082 | ATTATTTACAACGTCAC CG |
| | 106. | | | V3LHS_300078 | TTCTCGTCTAAAAGTGC GT |
| | 107. | | | V3LHS_300081 | AAAAGTGCGTGGTTGTC CG |
| | 108. | SAL L4 | Sal-like protein 4 Q9UJQ4 | V3LHS_363661 | TAGCTGACCGCAATCTT GT |
| | 109. | | | V3LHS_363659 | TAGTGAACTTCTTCTGG CA |
| | 110. | | | V3LHS_363662 | TCGGCTTGACTATTGGC CG |
| | 111. | | | V3LHS_363664 | TTCTGAGACTCTTTTTC CG |
| | 112. | SLC 25A 6 | ADP/ATP translocase 3 (gene SLC25A6) P12236 | V3LHS_314256 | TGTACTTATCCTTGAAG GC |
| | 113. | | | V3LHS_314257 | TGCCCGCAAAGTACCTC CA |
| | 114. | WD FY2 | WD repeat and FYVE domain-containing protein 2 Q96P53 | V2LHS_118254 | TATCCCACAACTTAATA AC |
| | 115. | | | V2LHS_118249 | TAACCAAACACGAACTG TC |
| | 116. | | | V3LHS_405758 | ATTGTATGAACAAGTTG GA |
| | 117. | | | V3LHS_341295 | TTCACAGGAGT CA TCTTGT |
| | 118. | | | V3LHS_405756 | TATATTGTATGAACAAG TT |
| | 119. | CB CP1 | cyclin Y | V2LHS_243158 | ATACTTGGCATAGACAC TG |
| | 120. | | | V3LHS_314369 | TACTGAGGAATATTGTG CT |
| | 121. | | | V3LHS_314371 | TAATGAAGAGACTCTTG CG |
| | 122. | MD M4 | | V2LHS_11941 | TATGTACTGACCTAAAT AG |
| | 123. | | | V2LHS_151660 | ATCTGAATACCAATCCT TC |
| | 124. | | | V3LHS_356802 | TGAACACTGAGCAGAG GTG |
| | 125. | | | V3LHS_356797 | AACAGTGAACATTTCAC CT |

**Table 2. MiniVector elements**

| **Table 2.** MiniVector elements | | | |
|---|---|---|---|
| Module | Element | Description | Use |
| A | λ-*att*L | *att*L from the λ-integrase system | Recombination sites (product of site-specific recombination used to generate MiniVector). Sequences listed in Table 3. |
| | λ*-att*R | *att*R from the λ-integrase system | |
| | λ*-att*B | *att*B from the λ-integrase system | |
| | λ*-att*P | *att*P from the λ-integrase system | |
| | *lox*P | *Iox*P site for Cre recombinase | |
| | γδ-*res* | *res* site for the γδ (Tn1000) resolvase | |
| | FRT | FRT site for FIp recombinase | |
| | *hixL* | *hix*L site for Hin recombinase | |
| | *hixR* | *hix*R site for Hin recombinase | |
| | Tn3 *res* | *res* site for Tn3 resolvase | |
| | Tn21 *res* | *res* site for Tn21 resolvase | |
| | *cer* | cer site for XerCD system | |
| | *psi* | *psi* site for XerCD | |
| B | ALDH1 | Tissue-specific promoter of alcohol dehydrogenase 1 (ALDH1) | Initiation of transcription. Includes promoters for RNA polymerase II and RNA polymerase III. Full sequences of selected promoters provided in Table 4. |
| | AMY1C | Tissue-specific promoter of human amylase alpha 1C (AMY1C) | |
| | β-actin | Promoter from the (human) beta actin gene | |
| | CaMKIIα | Ca2+/calmodulin-dependent protein kinase II alpha promoter | |
| | CMV | Promoter from the human cytomegalovirus (CMV) | |
| | Mini CMV | Minimized version of CMV | |
| | CAG | CMV early enhancer/chicken β actin promoter (CAG). Synthetic hybrid promoter made from a) the CMV early enhancer element, b) the promoter, the first exon and the first intron of chicken beta-actin gene, and c) the splice acceptor of the rabbit beta-globin gene | |
| | Cyto-keratin 18 and 19 | Cell-specific promoters of the human keratin 18 and 19 genes | |
| | EF1α | Strong expression promoter from human 1 alpha | |
| | GFAP | Tissue-specific promoter of the glial fibrillary acidic protein (GFAP) | |
| | H1h | Promoter from the human polymerase III RNA promoter | |
| | Kallikrein | Tissue-specific promoter of the kallikrein gene. | |
| | NFK-β | Nuclear factor kappa-light-chain-enhancer of activated B cells (NF-Kβ) | |
| | PGK1 | Promoter from human or mouse phosphoglycerate kinase gene (PGK) | |
| | RSV | Long terminal repeat (LTR) of the rous sarcoma virus (RSV) | |
| | SV40 | Mammalian expression promoter from the | |
| | | simian vacuolating virus 40 | |
| | UBC | Promoter of the human ubiquitin C gene (UBC) | |
| | U6 | Promoter from the human U6 small nuclear promoter | |
| C | shRNA | (DNA) sequence encoding short hairpin RNA (shRNA) transcript. Sequences for use in target validation are listed in Table 1. Potential therapeutic sequences will be designed *de novo* and optimized for knockdown efficiency. | Knockdown of gene expression through RNA interference |
| | miRNA | (DNA) sequence encoding micro-RNA (miRNA) transcript | |
| | lhRNA | (DNA) sequence encoding long hairpin RNA (IhRNA) transcript | |
| | lncRNA | (DNA) sequence encoding long non-coding RNA (lncRNA) transcript | Knockdown of gene expression (not RNAi) |
| | piRNA | (DNA) sequence encoding piwi-interacting (piRNA) RNA transcript | |
| D | Terminato r | Transcriptional terminator sequence | |
| E | S/MAR | Scaffold/matrix attached region from eukaryotic chromosomes (Sequences in Table 5) | Episomal replication |
| | CpG motifs | Unmethylated deoxycytidyl-deoxyguanosine (CpG) dinucleotides: (Sequences in Table 5) | Immunostimulat ory activity |
| F/G | β-globin intron | Intron of the human β globin gene (130 bp) | Gene expression enhancer |
| | HGH intron | Intron of the human growth hormone gene (262 bp) | |
| H | SV40 early promoter | Simian virus 40 early promoter (351 bp) | Nuclear localization |
| | NF-κβ | Binding site of nuclear factor kappa-light-chain-enhancer of activated B cells (55 bp (5 repeats of GGGGACTTTCC SEQ ID NO 159)) | |
| | p53 NLS | Binding site of tumor protein 53 (p53): AGACTGGGCATGTCTGGGCA SEQ ID NO 160 | |
| | p53 NLS | Binding site of tumor protein 53 (p53): GAACATGTCCCAACATGTTG SEQ ID NO 161 | |
| | Adenovirus major late promoter | GGGGCTATAAAAGGG SEQ ID NO 162 | |

**Table 3. Complete sequences for element A (recombination sites underlined)**

| SEQ ID NO | Site | Sequence (5'-3') |
|---|---|---|
| 126. | λ-*att*L | TCCGTTGAAGCCTGCTTTTTTATACTAAGTTGGCATTATA AAAAAGCATTGCTTATCAATTTGTTGCAACGAACAGGTCA CTATCAGTCAAAATAAAATCATTATT |
| 127. | λ-*att*R | AGATGCCTCAGCTCTGTTACAGGTCACTAATACCATCTA AGTAGTTGATTCATAGTGACTGCATATGTTGTGTTTTACA GTATTATGTAGTCTGTTTTTTATGCAAAATCTAATTTAATA TATTGATATTTATATCATTTTACGTTTCTCGTTCAGCTTTT TTATACTAACTTGAGCGAAACG |
| 128. | λ-*att*B | TCCGTTGAAGCCTGCTTTTTTATACTAACTTGAGCGAAAC G |
| 129. | λ-*att*P | AGATGCCTCAGCTCTGTTACAGGTCACTAATACCATCTA AGTAGTTGATTCATAGTGACTGCATATGTTGTGTTTTACA GTATTATGTAGTCTGTTTTTTATGCAAAATCTAATTTAATA TATTGATATTTATATCATTTTACGTTTCTCGTTCAGCTTTT TTATACTAAGTTGGCATTATAAAAAAGCATTGCTTATCAA TTTGTTGCAACGAACAGGTCACTATCAGTCAAAATAAAAT CATTATT |
| *130.* | *lox*P | ATAACTTCGTATAGCATACATTATACGAAGTTAT |
| 131. | γδ-*res* | ATTTTGCAACCGTCCGAAATATTATAAATTATCGCACACA TAAAAACAGTGCTGTTAATGTGTCTATTAAATCGATTTTTT GTTATAACAGACACTGCTTGTCCGATATTTGATTTAGGAT ACATTTTTA |
| 132. | FRT | GAAGTTCCTATTCTCTAGAAAGTATAGGAACTTC |
| 133. | *hixL* | TTCTTGAAAACCAAGGTTTTTGATAA |
| *134.* | *hixR* | TTTTCCTTTTGGAAGGTTTTTGATAA |
| 135. | Tn3 *res* | CAACCGTTCGAAATATTATAAATTATCAGACATAGTAAAA CGGCTTCGTTTGAGTGTCCATTAAATCGTCATTTTGGCAT AATAGACACATCGTGTCTGATATTCGATTTAAGGTACATT T |
| 136. | Tn21 *res* | GCCGCCGTCAGGTTGAGGCATACCCTAACCTGATGTCA GATGCCATGTGTAAATTGCGTCAGGATAGGATTGAATTT TGAATTTATTGACATATCTCGTTGAAGGTCATAGAGTCTT CCCTGACAT |
| 137. | cer | GGTGCGTACAATTAAGGGATTATGGTAAAT |
| 138. | psi | GGTGCGCGCAAGATCCATTATGTTAAAC |

**Table 4. Complete sequences for element B (promoters)**

| SEQ ID NO | Promoter | Sequence (5'-3') |
|---|---|---|
| 139. | CMV | GACATTGATTATTGACTAGTTATTAATAGTAATCAATTACGGGG TCATTAGTTCATAGCCCATATATGGAGTTCCGCGTTACATAACT TACGGTAAATGGCCCGCCTGGCTGACCGCCCAACGACCCCC GCCCATTGACGTCAATAATGACGTATGTTCCCATAGTAACGCC AATAGGGACTTTCCATTGACGTCAATGGGTGGAGTATTTACGG TAAACTGCCCACTTGGCAGTACATCAAGTGTATCATATGCCAA GTACGCCCCCTATTGACGTCAATGACGGTAAATGGCCCGCCT GGCATTATGCCCAGTACATGACCTTATGGGACTTTCCTACTTG GCAGTACATCTACGTATTAGTCATCGCTATTACCATGGTGATG CGGTTTTGGCAGTACATCAATGGGCGTGGATAGCGGTTTGAC TCACGGGGATTTCCAAGTCTCCACCCCATTGACGTCAATGGG AGTTTGTTTTGGCACCAAAATCAACGGGACTTTCCAAAATGTC GTAACAACTCCGCCCCATTGACGCAAATGGGCGGTAGGCGTG TACGGTGGGAGGTCTATATAAGCAGAGCT |
| 140. | mini-CMV | CCAAAATCAACGGGACTTTCCAAAATGTCGTAACAACTCCGCC CCATTGACGCAAATGGGCGGTAGGCGTGTACGGTGGGAGGT CTATATAAGCAGAGCT |
| 141. | RSV | GGTGCACACCAATGTGGTGAATGGTCAAATGGCGTTTATTGTA TCGAGCTAGGCACTTAAATACAATATCTCTGCAATGCGGAATT CAGTGGTTCGTCCAATCCATGTCAGACCCGTCTGTTGCCTTCC TAATAAGGCACGATCGTACCACCTTACTTCCACCAATCGGCAT GCACGGTGCTTTTTCTCTCCTTGTAAGGCATGTTGCTAACTCA TCGTTACCATGTTGCAAGACTACAAGAGTATTGCATAAGACTA CATT |
| 142. | CAG | GCGTTACATAACTTACGGTAAATGGCCCGCCTGGCTGACCGC CCAACGACCCCCGCCCATTGACGTCAATAATGACGTATGTTCC CATAGTAACGCCAATAGGGACTTTCCATTGACGTCAATGGGTG GAGTATTTACGGTAAACTGCCCACTTGGCAGTACATCAAGTGT ATCATATGCCAAGTACGCCCCCTATTGACGTCAATGACGGTAA ATGGCCCGCCTGGCATTATGCCCAGTACATGACCTTATGGGA CTTTCCTACTTGGCAGTACATCTACGTATTAGTCATCGCTATTA CCATGGTCGAGGTGAGCCCCACGTTCTGCTTCACTCTCCCCA TCTCCCCCCCCTCCCCACCCCCAATTTTGTATTTATTTATTTTT TAATTATTTTGTGCAGCGATGGGGGCGGGGGGGGGGGGGGG GCGCGCGCCAGGCGGGGCGGGGCGGGGCGAGGGGCGGGG CGGGGCGAGGCGGAGAGGTGCGGCGGCAGCCAATCAGAGC GGCGCGCTCCGAAAGTTTCCTTTTATGGCGAGGCGGCGGCG GCGGCGGCCCTATAAAAAGCGAAGCGCGCGGCGGGCG |
| 143. | EF1a | GCTCCGGTGCCCGTCAGTGGGCAGAGCGCACATCGCCCACA GTCCCCGAGAAGTTGGGGGGAGGGGTCGGCAATTGAACCGG TGCCTAGAGAAGGTGGCGCGGGGTAAACTGGGAAAGTGATGT CGTGTACTGGCTCCGCCTTTTTCCCGAGGGTGGGGGAGAACC GTATATAAGTGCAGTAGTCGCCGTGAACGTTCTTTTTCGCAAC GGGTTTGCCGCCAGAACACAGGTAAGTGCCGTGTGTGGTTCC CGCGGGCCTGGCCTCTTTACGGGTTATGGCCCTTGCGTGCCT TGAATTACTTCCACGCCCCTGGCTGCAGTACGTGATTCTTGAT CCCGAGCTTCGGGTTGGAAGTGGGTGGGAGAGTTCGAGGCC TTGCGCTTAAGGAGCCCCTTCGCCTCGTGCTTGAGTTGAGGC CTGGCCTGGGCGCTGGGGCCGCCGCGTGCGAATCTGGTGGC ACCTTCGCGCCTGTCTCGCTGCTTTCGATAAGTCTCTAGCCAT TTAAAATTTTTGATGACCTGCTGCGACGCTTTTTTTCTGGCAAG ATAGTCTTGTAAATGCGGGCCAAGATCTGCACACTGGTATTTC |
| | | GGTTTTGGGCCGCGGCGGCGACGGGGCCCGTGCGTCC CAGCGCACATGTTCGGCGAGGCGGGGCCTGCGAGCGCGGC CACCGAGAATCGGACGGGGGTAGTCTCAAGCTGGCCGGCCT GCTCTGGTGCCTGGCCTCGCGCCGCCGTGTATCGCCCCGCC CTGGGCGGCAAGGCTGGCCCGGTCGGCACCAGTTGCGTGAG CGGAAAGATGGCCGCTTCCCGGCCCTGCTGCAGGGAGCTCA AAATGGAGGACGCGGCGCTCGGGAGAGCGGGCGGGTGAGT CACCCACACAAAGGAAAAGGGCCTTTCCGTCCTCAGCCGTCG CTTCATGTGACTCCACGGAGTACCGGGCGCCGTCCAGGCACC TCGATTAGTTCTCGAGCTTTTGGAGTACGTCGTCTTTAGGTTG GGGGGAGGGGTTTTATGCGATGGAGTTTCCCCACACTGAGTG GGTGGAGACTGAAGTTAGGCCAGCTTGGCACTTGATGTAATT CTCCTTGGAATTTGCCCTTTTTGAGTTTGGATCTTGGTTCATTC TCAAGCCTCAGACAGTGGTTCAAAGTTTTTTTCTTCCATTTCAG GTGTCGTGA |
| 144. | EFS | ATCGATTGGCTCCGGTGCCCGTCAGTGGGCAGAGCGCACATC GCCCACAGTCCCCGAGAAGTTGGGGGGAGGGGTCGGCAATT GAACCGGTGCCTAGAGAAGGTGGCGCGGGGTAAACTGGGAA AGTGATGTCGTGTACTGGCTCCGCCTTTTTCCCGAGGGTGGG GGAGAACCGTATATAAGTGCAGTAGTCGCCGTGAACGTTCTTT TTCGCAACGGGTTTGCCGCCAGAACACAGGTGTCGTGACGCG |
| 145. | Human β-actin | GGCCTCCGCGCCGGGTTTTGGCGCCTCCCGCGGGCGCCCCC CTCCTCACGGCGAGCGCTGCCACGTCAGACGAAGGGCGCAG CGAGCGTCCTGATCCTTCCGCCCGGACGCTCAGGACAGCGG CCCGCTGCTCATAAGACTCGGCCTTAGAACCCCAGTATCAGC AGAAGGACATTTTAGGACGGGACTTGGGTGACTCTAGGGCAC TGGTTTTCTTTCCAGAGAGCGGAACAGGCGAGGAAAAGTAGT CCCTTCTCGGCGATTCTGCGGAGGGATCTCCGTGGGGCGGT GAACGCCGATGATTATATAAGGACGCGCCGGGTGTGGCACAG CTAGTTCCGTCGCAGCCGGGATTTGGGTCGCGGTTCTTGTTT GTGGATCGCTGTGATCGTCACTTGGTGAGTAGCGGGCTGCTG GGCTGGCCGGGGCTTTCGTGGCCGCCGGGCCGCTCGGTGG GACGGAAGCGTGTGGAGAGACCGCCAAGGGCTGTAGTCTGG GTCCGCGAGCAAGGTTGCCCTGAACTGGGGGTTGGGGGGAG CGCAGCAAAATGGCGGCTGTTCCCGAGTCTTGAATGGAAGAC GCTTGTGAGGCGGGCTGTGAGGTCGTTGAAACAAGGTGGGG GGCATGGTGGGCGGCAAGAACCCAAGGTCTTGAGGCCTTCG CTAATGCGGGAAAGCTCTTATTCGGGTGAGATGGGCTGGGGC ACCATCTGGGGACCCTGACGTGAAGTTTGTCACTGACTGGAG AACTCGGTTTGTCGTCTGTTGCGGGGGCGGCAGTTATGGCGG TGCCGTTGGGCAGTGCACCCGTACCTTTGGGAGCGCGCGCC CTCGTCGTGTCGTGACGTCACCCGTTCTGTTGGCTTATAATGC AGGGTGGGGCCACCTGCCGGTAGGTGTGCGGTAGGCTTTTC TCCGTCGCAGGACGCAGGGTTCGGGCCTAGGGTAGGCTCTC CTGAATCGACAGGCGCCGGACCTCTGGTGAGGGGAGGGATA AGTGAGGCGTCAGTTTCTTTGGTCGGTTTTATGTACCTATCTT CTTAAGTAGCTGAAGCTCCGGTTTTGAACTATGCGCTCGGGG TTGGCGAGTGTGTTTTGTGAAGTTTTTTAGGCACCTTTTGAAAT GTAATCATTTGGGTCAATATGTAATTTTCAGTGTTAGACTAGTA AATTGTCCGCTAAATTCTGGCCGTTTTTGGCTTTTTTGTTAGAC |
| 146. | NFK-β | GCTAGCGGGAATTTCCGGGAATTTCCGGGAATTTCCGGGAAT TTCCAGATCTGCCGCCCCGACTGCATCTGCGTGTTCGAATTC GCCAATGACAAGACGCTGGGCGGGGTTTGTGTCATCATAGAA CTAAAGACATGCAAATATATTTCTTCCGGGGACACCGCCAGCA AACGCGAGCAACGGGCCACGGGGATGAAGCAGAAGCTTGGC A |
| 147. | Ubiquitin-C | GTCTAACAAAAAAGCCAAAAACGGCCAGAATTTAGCGGACAAT TTACTAGTCTAACACTGAAAATTACATATTGACCCAAATGATTA CATTTCAAAAGGTGCCTAAAAAACTTCACAAAACACACTCGCC AACCCCGAGCGCATAGTTCAAAACCGGAGCTTCAGCTACTTAA GAAGATAGGTACATAAAACCGACCAAAGAAACTGACGCCTCA CTTATCCCTCCCCTCACCAGAGGTCCGGCGCCTGTCGATTCA GGAGAGCCTACCCTAGGCCCGAACCCTGCGTCCTGCGACGG AGAAAAGCCTACCGCACACCTACCGGCAGGTGGCCCCACCCT GCATTATAAGCCAACAGAACGGGTGACGTCACGACACGACGA GGGCGCGCGCTCCCAAAGGTACGGGTGCACTGCCCAACGGC ACCGCCATAACTGCCGCCCCCGCAACAGACGACAAACCGAGT TCTCCAGTCAGTGACAAACTTCACGTCAGGGTCCCCAGATGG TGCCCCAGCCCATCTCACCCGAATAAGAGCTTTCCCGCATTA GCGAAGGCCTCAAGACCTTGGGTTCTTGCCGCCCACCATGCC CCCCACCTTGTTTCAACGACCTCACAGCCCGCCTCACAAGCG TCTTCCATTCAAGACTCGGGAACAGCCGCCATTTTGCTGCGCT CCCCCCAACCCCCAGTTCAGGGCAACCTTGCTCGCGGACCCA GACTACAGCCCTTGGCGGTCTCTCCACACGCTTCCGTCCCAC CGAGCGGCCCGGCGGCCACGAAAGCCCCGGCCAGCCCAGC AGCCCGCTACTCACCAAGTGACGATCACAGCGATCCACAAAC AAGAACCGCGACCCAAATCCCGGCTGCGACGGAACTAGCTGT GCCACACCCGGCGCGTCCTTATATAATCATCGGCGTTCACCG CCCCACGGAGATCCCTCCGCAGAATCGCCGAGAAGGGACTA CTTTTCCTCGCCTGTTCCGCTCTCTGGAAAGAAAACCAGTGCC CTAGAGTCACCCAAGTCCCGTCCTAAAATGTCCTTCTGCTGAT ACTGGGGTTCTAAGGCCGAGTCTTATGAGCAGCGGGCCGCTG TCCTGAGCGTCCGGGCGGAAGGATCAGGACGCTCGCTGCGC CCTTCGTCTGACGTGGCAGCGCTCGCCGTGAGGAGGGGGGC GCCCGCGGGAGGCGCCAAAACCCGGCGCGGAGGC |
| 148. | SV40 | GGTGTGGAAAGTCCCCAGGCTCCCCAGCAGGCAGAAGTATG CAAAGCATGCATCTCAATTAGTCAGCAACCAGGTGTGGAAAGT CCCCAGGCTCCCCAGCAGGCAGAAGTATGCAAAGCATGCATC TCAATTAGTCAGCAACCATAGTCCCGCCCCTAACTCCGCCCAT CCCGCCCCTAACTCCGCCCAGTTCCGCCCATTCTCCGCCCCA TGGCTGACTAATTTTTTTTATTTATGCAGAGGCCGAGGCCGCC TCGGCCTCTGAGCTATTCCAGAAGTAGTGAGGAGGCTTTTTTG GAGGCCTAGGCTTTTGCAAA |
| 149. | PGK | CCGGTAGGCGCCAACCGGCTCCGTTCTTTGGTGGCCCCTTCG CGCCACCTTCTACTCCTCCCCTAGTCAGGAAGTTCCCCCCCG CCCCGCAGCTCGCGTCGTGCAGGACGTGACAAATGGAAGTA GCACGTCTCACTAGTCTCGTGCAGATGGACAGCACCGCTGAG CAATGGAAGCGGGTAGGCCTTTGGGGCAGCGGCCAATAGCA GCTTTGCTCCTTCGCTTTCTGGGCTCAGAGGCTGGGAAGGGG TGGGTCCGGGGGCGGGCTCAGGGGCGGGCTCAGGGGCGGG GCGGGCGCCCGAAGGTCCTCCGGAGGCCCGGCATTCTGCAC GCTTCAAAAGCGCACGTCTGCCGCGCTGTTCTCCTCTTCCTCA TCTCCGGGCCTTTCGACCTGCAGCC |
| 150. | H1 | AATATTTGCATGTCGCTATGTGTTCTGGGAAATCACCATAAAC GTGAAATGTCTTTGGATTTGGGAATCTTATAAGTTCTGTATGAG ACCACAGATCCC |
| 151. | U6 | GATCCGACGCCGCCATCTCTAGGCCCGCGCCGGCCCCCTCG CACAGACTTGTGGGAGAAGCTCGGCTACTCCCCTGCCCCGGT TAATTTGCATATAATATTTCCTAGTAACTATAGAGGCTTAATGT GCGATAAAAGACAGATAATCTGTTCTTTTTAATACTAGCTACAT TTTACATGATAGGCTTGGATTTCTATAAGAGATACAAATACTAA ATTATTATTTTAAAAAACAGCACAAAAGGAAACTCACCCTAACT GTAAAGTAATTGTGTGTTTTGAGACTATAAATATCCCTTGGAGA AAAGCCTTGTT |

**Table 5. Complete sequences for elements E, F and G (accessory sequences)**

| SEQ ID NO | Element | Sequence (5'-3') |
|---|---|---|
| 152. | 250 bp S/MAR | TCTTTAATTTCTAATATATTTAGAATCTTTAATTTCTAATAT ATTTAGAATCTTTAATTTCTAATATATTTAGAATCTTTAAT TTCTAATATATTTAGAATCTTTAATTTCTAATATATTTAGA ATCTTTAATTTCTAATATATTTAGAATCTTTAATTTCTAAT ATATTTAGAATCTTTAATTTCTAATATATTTAGAATCTTTA ATTTCTAATATATTTAGAATCTTTAATTTCTAATATATTTA GAA |
| 153. | 439 bp S/MAR | TCTTTAATTTCTAATATATTTAGAATCTTTAATTTCTAATAT ATTTAGAATCTTTAATTTCTAATATATTTAGAATCTTTAAT TTCTAATATATTTAGAATCTTTAATTTCTAATATATTTAGA ATCTTTAATTTCTAATATATTTAGAATCTTTAATTTCTAAT ATATTTAGAATCTTTAATTTCTAATATATTTAGAATCTTTA ATTTCTAATATATTTAGAATCTTTAATTTCTAATATATTTA GAA |
| 154. | (45 bp) Type A CpG motif | GGTGCATCGATGCAGCATCGAGGCAGGTGCATCGATAC AGGGGGG |
| 155. | (24 bp) Type B CpG motif | TCGTCGTTTTGTCGTTTTGTCGTT |
| 156. | (21 bp) Type C CpG motif | TCGTCGAACGTTCGAGATGAT |
| 157. | β-globin intron | GTTGGTATCAAGGTTACAAGACAGGTTTAAGGAGACCAA TAGAAACTGGGCATGTGGAGACAGAGAAGACTCTTGGG TTTCTGATAGGCACTGACTCTCTCTGCCTATTGGTCTATT TTCCCACCCTTAG |
| 158. | Human growth hormone intron | TTCGAACAGGTAAGCGCCCCTAAAATCCCTTTGGGCAC AATGTGTCCTGAGGGGAGAGGCAGCGACCTGTAGATGG GACGGGGGCACTAACCCTCAGGTTTGGGGCTTCTGAAT GTGAGTATCGCCATGTAAGCCCAGTATTTGGCCAATCTC AGAAAGCTCCTGGTCCCTGGAGGGATGGAGAGAGAAAA ACAAACAGCTCCTGGAGCAGGGAGAGTGCTGGCCTCTT GCTCTCCGGCTCCCTCTGTTGCCCTCTGGTTTC |

Each of the following references are incorporated by reference in their entirety for all purposes.

Catanese, D.J., et al., Supercoiled MiniVector DNA resists shear forces associated with gene therapy delivery, Gene Ther. 19(1): 94-100 (2012).

Darquet A.M., et al., Minicircle: an improved DNA molecule for in vitro and in vivo gene transfer, Gene Ther. 6: 209-218 (1999).

Fogg, J.M., et al., Exploring writhe in supercoiled minicircle DNA. J. Phys.-Condes. Matter. 18: S145-S159 (2006).

Hardee, C.L., Advances in Non-Viral DNA Vectors for Gene Therapy, Genes. 8, 65 (2017)

Hornstein, B.D., et al., Effects of Circular DNA Length on Transfection Efficiency by Electroporation into HeLa Cells, PLoS One. 11(12): e0167537 (2016).

Lis and Schleif, Size fractionation of double-stranded DNA by precipitation with polyethylene glycol. Nucleic Acids Research. 2, 383-389 (1975).

US20150376645, US20140056868, 61/653,279, filed May 30, 2012, Supercoiled MiniVectors as a tool for DNA repair, alteration and replacement

US8460924, US8729044, US9267150, US20110160284, US20120302625, US20130316449, 61/252,455, filed Oct. 16, 2009, Supercoiled MiniVectors for gene therapy applications

US7622252, US20070020659, 60/689,298, filed Jun. 10, 2005, Generation of minicircle DNA with physiological supercoiling

US20060211117 Methods of making minicircles

WO1994009127 Supercoiled minicircle DNA as a unitary promoter vector

WO2002083889 Methods for the production of minicircles

Ramamoorth M., & Narvekar, A., Non Viral Vectors in Gene Therapy- An Overview, J. Clinical & Diagnostic Res. 2015 Jan, Vol-9(1): GE01-GE06.

Hidai C., & Kitano, H., Nonviral Gene Therapy for Cancer: A Review, Diseases. 2018, 6, 57.

## Claims

1. A composition comprising an ultrapure MiniVector plus a pharmaceutically acceptable carrier,
a) said MiniVector being a double-stranded, supercoiled circular DNA lacking a bacterial origin of replication and lacking an antibiotic selection gene or any other plasmid selection marker;
b) an expressable payload sequence;
c) said MiniVector being 100-1000 bp in length excluding a length of said payload sequence; and
d) said MiniVector having < 0.02% contamination by a parent plasmid DNA or recombination side-products.

2. The ultrapure MiniVector of claim 1, wherein contamination is assessed by gel electrophoresis and staining at a sensitivity of ≤ 0.1 ng, or preferably ≤ 0.01 ng.

3. The ultrapure MiniVector of claim 1, wherein contamination is assessed by gel electrophoresis and staining with SYBR Gold staining at a sensitivity of ≤ 0.1 ng.

4. The ultrapure MiniVector of claim 1, wherein contamination is assessed by gel electrophoresis, Southern blotting and probing with radiolabeled sequences that are unique to said parent DNA staining at a sensitivity of ≤ 0.01 ng.

5. The ultrapure MiniVector of claim 1, wherein said MiniVector is separated from said parent plasmid and recombination side-products on the basis of size, and does not use sequence-specific endonuclease cleavage *in vivo* for preparation of said MiniVector.

6. The ultrapure MiniVector of claim 1, wherein said MiniVector is purified by PEG precipitation of large DNA and at least two passes through multiple gel filtration columns containing different size exclusion resins, each covering a different molecular weight size range.

7. The ultrapure MiniVector of claim 1, wherein said MiniVector is purified by PEG precipitation of larger DNA species followed by anion exchange chromatography to remove RNA and non-nucleic acid components, followed by at least two passes through multiple gel filtration columns containing different size exclusion resins, each covering a different molecular weight size range.

8. The ultrapure MiniVector of claim 1, wherein said MiniVector is purified by PEG precipitation, anion exchange chromatography, and at least two passes through multiple gel filtration columns containing different size exclusion resins, each covering a different molecular weight size range, and one or more alcohol precipitations.

9. The ultrapure MiniVector of claim 1, further comprising a promoter operably connected to said payload sequence operably connected to a terminator.

10. The ultrapure MiniVector of claim 1, wherein said payload sequence encodes:
a) an inhibitory RNA for a target gene selected from FOXM1, AKT, CENPA, PLK1, CDC20, BIRC5, AURKB, CCNB1, CDKN3, BCAM-AKT2, CDKN2D-WDFY2, SLC25A6, CIP2A, CD133, ALDH1A1, CD44, SALL4, CHD11, MDM2, MDM4 and/or PRDM16, alone, or in combination, and wherein expression of said target gene is reduced at least 10% by said inhibitory RNA when said MiniVector is introduced into mammalian cells and expressed therein; or
b) an apoptosis gene selected from p53, p63, p73, p16, p21, p27, E2F genes, FHIT, PTEN, and/or CASPASE alone, or in combination, and said apoptosis gene is overexpressed when said MiniVector is introduced into mammalian cells.

11. The MiniVector of claim 1, wherein said payload sequence encodes an inhibitory RNA for a target gene selected from FOXM1.

12. A composition comprising a MiniVector in a pharmaceutically acceptable excipient, said MiniVector being a double-stranded circular DNA encoding an expressible payload sequence and lacking a bacterial origin of replication and lacking an antibiotic resistance gene or plasmid selection marker, wherein said MiniVector is at least 99.98% free of parent plasmid DNA or recombination side-products, wherein said payload is expressible in human cells and thereby inhibits the expression of a human target gene selected from FOXM1, AKT, CENPA, PLK1, CDC20, BIRC5, AURKB, CCNB1, CDKN3, BCAM-AKT2, CDKN2D-WDFY2, SLC25A6, CIP2A, CD133, ALDH1A1, CD44, SALL4, MDM2, MDM4, and/or PRDM16, alone, or in any combination.

13. The composition of claim 12, wherein said MiniVector is <350 bp in length, excluding said payload sequence; or
wherein said MiniVector is <100 bp in length, excluding said payload sequence.

14. The composition of claim 12, wherein said MiniVector is CpG-minimized or CpG free by replacing one or more CpG dinucleotides in the MiniVector sequence; or
wherein said MiniVector is supercoiled; or
wherein said MiniVector has a specific DNA sequence-defined shape.

15. A MiniVector, said MiniVector being a double-stranded, supercoiled, circular DNA of at least 99.98% purity from contaminating parent plasmid DNA or recombination side products and encoding a payload that can be expressed in a mammalian cell, wherein said payload encodes an inhibitory RNA for a target gene selected from FOXM1, AKT, CENPA, PLK1, CDC20, BIRC5, AURKB, CCNB1, CDKN3, BCAM-AKT2, CDKN2D-WDFY2, SLC25A6, CIP2A, CD133, ALDHIA1, CD44, SALL4, MDM2, MDM4, and/or PRDM16, alone or in any combination, wherein said MiniVector lacks a bacterial origin of replication and lacks an antibiotic resistance gene or plasmid selection marker, and wherein said MiniVector is made by:
a) engineering a parent plasmid DNA molecule comprising site-specific recombination sites on either side of said expressible payload;
b) transforming said parent plasmid into a cell suitable for site-specific recombination to occur, under conditions such that topoisomerase IV decatenation activity is inhibited, thereby producing a plurality of catenated DNA circles, wherein at least one of the circles in each catenane is a supercoiled MiniVector of less than about 2 kb in length;
c) decatenating the catenated site-specific recombination products, thereby releasing the supercoiled MiniVector from the catenanes; and
d) isolating the supercoiled MiniVector by PEG precipitation, anion exchange and at least two size exclusion resins each covering a different size range such that said MiniVector is at least 99.98% pure of parent plasmid or recombination side products.
